(19) **Europäisches Patentamt / European Patent Office / Office européen des brevets**

(11) **EP 4 489 718 B1**

(12) **EUROPEAN PATENT SPECIFICATION**

(45) Date of publication and mention
of the grant of the patent:
**11.03.2026 Bulletin 2026/11**

(21) Application number: **23708796.0**

(22) Date of filing: **03.03.2023**

(51) International Patent Classification (IPC):
*A61K 8/02* (2006.01)    *A61K 8/67* (2006.01)
*A61Q 19/08* (2006.01)    *A61K 8/49* (2006.01)
*A61K 8/55* (2006.01)    *A61K 8/60* (2006.01)
*A61K 8/92* (2006.01)    *A61K 8/11* (2006.01)

(52) Cooperative Patent Classification (CPC):
**A61K 8/671; A61K 8/11; A61K 8/4973;
A61K 8/553; A61K 8/604; A61K 8/92; A61K 8/922;
A61Q 19/08;** A61K 2800/412

(86) International application number:
**PCT/EP2023/055480**

(87) International publication number:
**WO 2023/169963 (14.09.2023 Gazette 2023/37)**

(54) **METHOD FOR ENCAPSULATING A HYDROPHOBIC COMPOUND, SUSPENSION OF STRUCTURED LIPID PARTICLES ACCORDING TO THIS METHOD, AND USES OF SAID SUSPENSION**

VERFAHREN ZUR EINKAPSELUNG EINER HYDROPHOBEN VERBINDUNG, SUSPENSION STRUKTURIERTER LIPIDPARTIKEL NACH DIESEM VERFAHREN UND VERWENDUNGEN DIESER SUSPENSION

PROCÉDÉ D'ENCAPSULATION D'UN COMPOSÉ HYDROPHOBE, SUSPENSION DE PARTICULES LIPIDIQUES STRUCTURÉES SELON CE PROCÉDÉ, ET UTILISATIONS DE LADITE SUSPENSION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **07.03.2022 FR 2201971**

(43) Date of publication of application:
**15.01.2025 Bulletin 2025/03**

(73) Proprietor: **SEDERMA**
**78610 Le-Perray-en-Yvelines (FR)**

(72) Inventors:
• **FOURNIAL, Arnaud**
**75016 PARIS (FR)**
• **PLATCHECK RAFFIN, Renata**
**91530-008 PORTO ALEGRE (BR)**
• **ROSSINI KOPP, Cristieli**
**95555-000 RIO GRANDE DO SUL (BR)**

(74) Representative: **de Saint Viance, Isabelle Marie Fanny**
**Sederma**
**29, rue du chemin vert**
**78610 Le-Perray-en-Yvelines (FR)**

(56) References cited:
**EP-A1- 1 428 571**    **EP-A1- 2 047 838**
**US-A1- 2008 089 913**

Remarks:
The complete document including Reference Table(s) and the Sequence Listing(s) can be downloaded from the EPO website

**Description**

<u>TECHNICAL FIELD</u>

The present invention relates to a method for encapsulating a hydrophobic compound or a mixture of hydrophobic compounds, in particular one or more biologically active molecules, such as retinol, an aqueous suspension of structured lipid particles that can be obtained according to this method, and uses of said suspension, in particular for a non-therapeutical cosmetic treatment of the skin and/or its appendages (such as body hair, eyelashes, eyebrows, nails, hair) of mammals, humans or animals, and for manufacturing a topical or oral composition intended in particular to this treatment. The present invention also relates to the dried form (or powder) of the aqueous suspension.

The present invention concerns the field of cosmetic, pharmaceutical, veterinary products, food supplements, hygiene, and personal care products, topically or orally.

<u>BACKGROUND ART</u>

Retinol (or vitamin A) is one of the best known and widely used cosmetic active ingredients to treat photo-aging of skin and its appendages. It is a twenty-carbon molecule, comprising a cyclohexenyl ring with a side chain with four double bonds all in trans configuration and a terminal alcohol group, hence its name also of all-trans retinol. Oxidation of the terminal alcohol group in retinol results in the formation of an aldehyde (all-trans retinaldehyde or retinal), which can then be oxidized to a carboxylic acid (all-trans retinoic acid or tretinoin). Retinol has the major disadvantage of being very labile. Its degradation occurs very quickly and makes it difficult to formulate stable cosmetic products. The derivatives of retinaldehydes and retinoic acid are less active than retinol and more aggressive for the skin. In addition, they colour cosmetic formulations in yellow, an obvious sign of product degradation for the consumer.

Since retinol is a lipophilic active ingredient, it is often offered dissolved in oil, especially soybean oil, and combined with an antioxidant. The oil will protect the retinol but not enough. Degradation is only delayed, and handling and storage constraints must be observed.

Encapsulation is one of the known strategies to overcome the stability problem of an active by protecting it inside a particle.

Lipid nanoparticles are well-suited encapsulation systems for hydrophobic/lipophilic molecules, such as retinol. These molecules can be dissolved in a lipid matrix which will then form the particles dispersed in an aqueous phase. Furthermore, in the case of topical use, penetration through the *stratum corneum* into deeper layers of the skin is facilitated thanks to a lipid rearrangement at the level of the *stratum corneum,* the lipid composition of the particles and their very small size (submicronic) contributing to increase the particle adhesion and their contact surface.

To encapsulate this type of active or a mixture of actives, historically, wax-based lipid nanoparticles were first proposed, solid at room temperature, namely the temperature of use on the skin, particles being called SLN for "solid lipid nanoparticles". The labile active molecule(s) are trapped in waxy particles, being distributed more or less homogeneously in these particles.

Second-generation lipid nanoparticles were thereafter proposed. These particles are prepared from two types of lipids: solid lipids and liquid lipids at room temperature. These particles are called SLC for "solid liquid nanocarrier". The mixture of these two types of lipids leads to a less crystalline particle, mixing oily zones and waxy zones. Thanks to this new type of particles, the hydrophobic labile molecule is better protected by a substantially waxy shell and is less likely to be expelled.

More recently, a new type of particles has been developed, called SmartLipids® developed by the BergaCare company. The lipid matrix consists of a mixture of about ten specific waxes and oils chosen to form at the end of the process at room temperature an unstructured lipid matrix, namely forming a matrix of structure identified as non-coherent (disorganized) of oils and waxes in which the hydrophobic labile molecule is trapped in an optimized manner. The encapsulation rate can be increased, but not necessarily the stability over time, which is still insufficient.

All these particles are, for example, described in the scientific publications "Lipid nanoparticles (SLN, NLC) in cosmetic and pharmaceutical dermal products", J. Pardeike et al. / International Journal of Pharmaceutics 366 (2009) 170-184, or more recently "BergaCare SmartLipids: concentrated commercial lipophilic active to improve the performance of dermal products", F. Olechowski et al., Beilstein J. Nanotechnol. 2019, 10, 2152-2162, retinol being one of the candidate labile actives for this type of particle.

To increase the stability over time, it has also been proposed to manufacture the particles (NLC or SmartLipids® type) with a high-pressure vacuum homogenization system to minimize the air trapped in the particles. Mainly, this method protects the trapped molecules from oxidation. Reference can be made to the following article which deals with encapsulation *via* this type of retinol particle: "Synthesis of lipid nanocarrier loaded with retinol via vacuum emulsification to improve topical delivery", Seung-Hyun Jun et al., Polymers 2021, 13, 826.

EP2047838 is related to the area of encapsulation and concerns new microcapsules based on waxes, a process for obtaining them and their application in various areas. The document discloses a tocopherol encapsulation process comprising a step of forming a mixture of an aqueous phase, a waxy phase (dibutyl adipate), an oily phase (behenyl alcohol), the mixture comprising a surfactant (polysorbate 20 EO).

More specifically, the present invention relates to a method for the manufacture of an aqueous suspension of lipid particles of the NLC type, namely substantially formed at room temperature, from a waxy phase and an oily phase.

The term "submicron particles" will be used according to the invention for particles of submicron average diameter and not the term "nanoparticles" because the particles according to the invention do not correspond to the definition of the WTO and the regulations of the European Union which limits the term "nanoparticles" to insoluble particles with a nominal diameter ranging from 1 to 100 nm.

The term "average" submicron diameter corresponds to the median diameter of the particles at the 50th percentile of the distribution curve.

The encapsulation method according to the invention is of the type comprising:

- a step of providing a mixture comprising an aqueous phase, a waxy phase, and an oily phase containing said at least one compound, the mixture further comprising one or more surfactants, and optionally each phase being made homogeneous and liquid by heating;
- a hot forming step of an emulsion of said mixture;
- a step of reducing the particle size of said emulsion substantially to a submicron size, for example by hot homogenization under high pressure; and
- a cooling step to form said suspension of submicron particles.

At room temperature (RT), namely at a temperature of use substantially between 15°C and 30°C, an aqueous suspension of submicron lipid particles is obtained, the hydrophobic compound or mixture of compounds being encapsulated in the particles, namely trapped inside the particles.

This method is advantageous. Industrialization is simple, organic solvents can be avoided and production times are short. High pressure homogenizers are widely used in many industries, including cosmetics and pharmaceuticals, to obtain submicron particles.

The choice of surfactants is one of the solutions to improve the method.

Commonly, surfactants are chosen with a high HLB (significantly greater than 10) to stabilize the formation of the particle shell and their size, and are mixed, alone or in combination, with the aqueous phase. The following are thus described, for example: Poloxamer® (non-ionic three-block copolymers, typically having a hydrophobic central block of polypropylene glycol and two hydrophilic outer blocks of polyethylene glycol (also called polyethylene oxide), polysorbates (esters of fatty acids and polyoxyethylene sorbitan), in particular Tween 20™ or Tween 60™, sodium stearoyl glutamate and Plantacare™ alkylglucosides, 818 UP (Coco-glucoside), 2000 UP (Decyl Glucoside) or 1200 UP (lauryl glucoside). In addition to the scientific publications mentioned above, mention may be made of the publication "Effect of the surfactant on nanostructured lipid carriers loaded with lycopene", Pornthida Riangjanapatee et al., Drug Discoveries & Therapeutics. 2012; 6(3):163-168.

The chemical nature of the surfactant(s), in particular the ionic, non-ionic, or amphoteric nature, can also influence the size of the particles and their distribution, as well as the polarity of the particles, for example for avoiding aggregation and phase separation during production or after a certain period.

Hydrogenated lecithin, having a low HLB, is also sometimes used as a surfactant/solubilizer for DNA or RNA lipid nanoparticles, as in the case of vaccines.

Other studies have been done on the influence of the wax/oil ratio. The ratios vary depending on the type of wax and oil used. The article by Apostolou et al., Journal of Pharmaceutical Sciences 110 (2021) 2859-2872. presents a recent review of this subject and describes a maximum wax/oil ratio of 1:1.5.

However, the results obtained to date are not yet satisfactory both on the formation of the particles themselves, the encapsulation rate of active molecule or mixture of active molecules, and on the stability of the encapsulation over time, while maintaining a submicron particle size suitable for good penetration into the skin. An encapsulation rate of at least 70% and a stability over time of at least 1 year are sought, in particular for the cosmetics industry.

SUMMARY OF THE INVENTION

The aim of the present invention is to overcome all these drawbacks, and in addition to meet the growing needs for "naturalness" of the raw materials used in the fields of cosmetics and pharmacy.

To this end, the present invention provides, according to a first aspect, an encapsulation method according to which an aqueous suspension of submicron structured lipid particles is formed, at least one hydrophobic compound being encapsulated in said particles, said method comprising:

- a step of forming a mixture comprising an aqueous phase, a waxy phase, and an oily phase containing said at least one compound, the mixture further comprising one or more surfactants, and each phase being made homogeneous and liquid, if necessary, optionally by heating;
- a step of hot forming of an emulsion of said mixture;
- a step of reducing the particle size of said emulsion substantially to the submicron size of said particles; and
- a cooling step to form said suspension of structured particles,

said method being characterized in that:

- the ratio of the % by weight of wax: % by weight of oil is from 1: 2 to 1: 15,
- the following system of three surfactants is used:

  - a first non-ionic surfactant (S1) having an HLB greater than or equal to 10;
  - a second surfactant (S2) which is solid at room temperature, which is an ester of sorbitan and saturated C12-C24 fatty acid and having an HLB of less than or equal to 6; and
  - a third surfactant (S3) which is a phospholipid,

all weight % being expressed relative to the total weight of the mixture or of the suspension.

All weight % are calculated at room temperature (as defined above).

This method is advantageously suitable for the encapsulation of hydrophobic vitamins, such as retinol, or tocopherol derivatives, coenzyme Q10, resveratrol, ferulic acid, ascorbic acid, lipoic acid or salicylic acid, and their derivatives.

Thanks to the specific feature combination according to the invention, in particular the choice of the wax/oil ratio, the specific system of three surfactants, in particular the fact that one of them is low HLB and solid at room temperature, as illustrated by the results of tests and studies given in the detailed below description, it is obtained with success, for example with a retinol encapsulation:

- spherical and homogeneous submicron particles at room temperature (namely between 15°C and 30°C), for example in the case of retinol following a Gaussian size profile around an average value of 300 nm, with low polydispersity, and possibly comprising a retinol content after encapsulation of 3.0 to 3.5%, entirely compatible with a use for formulating cosmetic products;
- "structured" particles meaning according to the invention that the particle has been identified substantially structured into a waxy shell and an oily core, unlike SLC-type particles having a more or less heterogeneous matrix, or completely heterogeneous such as the SmartLipids®;
- an aqueous suspension stable at room temperature for one year, maintaining the retinol content at more than 2.0%, without coloration, therefore without significant degradation and loss of activity;
- a storage which can advantageously be done at room temperature and handling under an air atmosphere,
- a protection of the encapsulated active against UV degradation;
- a release profile that is slow, with a half-life adapted to topical diffusion, meaning that the stability of the active is maintained during the release period in the skin;
- a possible choice of all raw materials that respect environmental and biodegradable constraints; and
- a demonstrated penetration of the encapsulated compound into the epidermis.

The detailed description given below illustrates in detail all these advantages as well as others obtained thanks to the method of the invention.

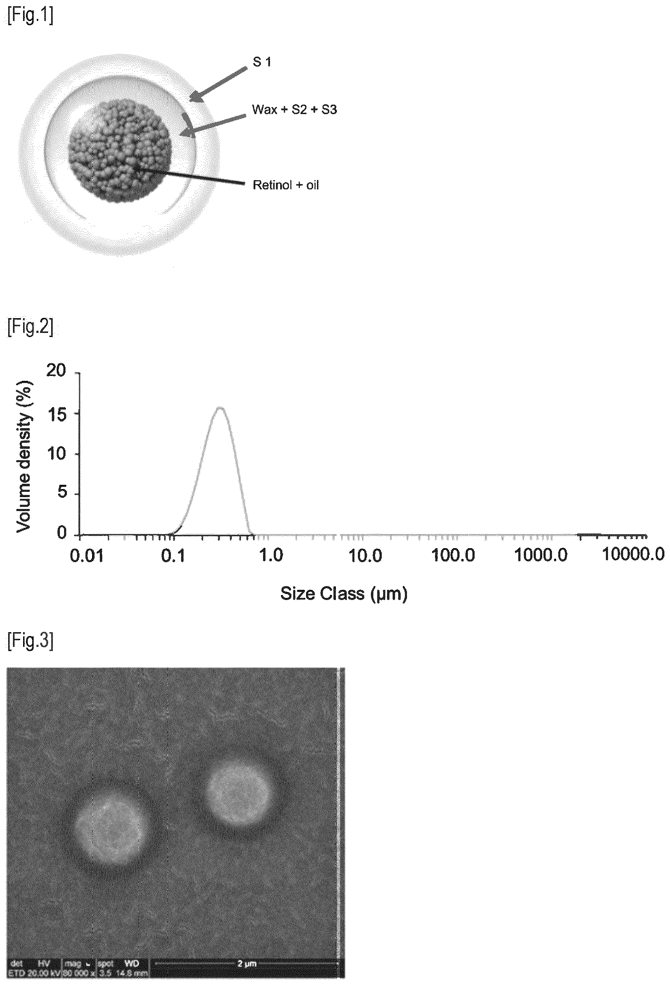

Figure 1 schematically illustrates the predictive structure of the particles obtained: a waxy shell surrounded by the first surfactant which stabilizes it, an oily core in which the active ingredient is solubilized (here retinol), the second and the third surfactants being associated with the waxy shell (see the detailed description below). According to preferred and advantageous features of the invention:

- the first surfactant is non-ethoxylated, and/or
- the first surfactant comprises at least one alkylglucoside or one alkylpolyglucoside of at least one saturated C10-C16 fatty alcohol, preferably chosen from lauryl glucoside, decyl glucoside, coco glucoside and their polyglucoside derivatives, more preferably lauryl glucoside; and/or
- the first surfactant is added to the aqueous phase, dissolved, if necessary, by heating; and/or
- the second surfactant is a monoester chosen from sorbitan monostearate, sorbitan sesquistearate, sorbitan laurate, sorbitan oleate, sorbitan sesquioleate and sorbitan isostearate, more preferably sorbitan isostearate sorbitan; and/or

- the second surfactant is added to the aqueous phase, dissolved by heating; and/or
- the third surfactant is chosen from phosphatidic acid, a phosphatidylcholine or lysophosphatidylcholine, a glycerophosphocholine, a phosphatidylserine or a lysophosphatidylserine, a phosphatidylethanolamine, a phosphatidylinositol or a sphingomyelin, more preferably the third surfactant being hydrogenated, and more preferentially being hydrogenated lecithin; and/or
- the third surfactant is added to the waxy phase, dissolved, if necessary, by heating; and/or
- a wax is used which is natural or synthetic, consisting of at least one ester of C12-C36 fatty acid(s) and of C12-C36 fatty alcohol(s), preferably chosen from triglycerides, diglycerides, monoglycerides, and monoesters of C18-C36 fatty acid(s) and of C18-C36 fatty alcohol(s), more preferably the cetyl palmitate, glyceryl tribehenate, stearate or tristearate of glyceryl, a wax of vegetable origin, such as rice wax, or a wax of animal origin, such as beeswax; and/or
- an oil is used which is formed of essentially C16-C18 fatty acid(s) or of mono-, di- or tri-glycerides of said fatty acid(s), preferably a vegetable oil, more preferably soy, rapeseed, sunflower, palm or coconut oil; and/or
- the percentage by weight of wax is from 0.5% to 10% relative to the total weight of the mixture or of the suspension, preferably from 1% to 6%, more preferably from 2% to 5%, and still more preferably from 2.5 to 4%; and/or
- the percentage by weight of oil is from 5% to 40% relative to the total weight of the mixture or of the suspension, preferably 10% to 35%, more preferably 20% to 30%; and/or
- the percentage by weight of the first surfactant is from 1% to 5% relative to the total weight of the mixture or of the suspension, preferably from 1% to 3%; and/or
- the percentage by weight of the second surfactant is from 0.1% to 5% relative to the total weight of the mixture or of the suspension, preferably from 0.1% to 3%, more preferably from 0.3% at 1%; and/or
- the percentage by weight of the third surfactant is from 0.1% to 2% relative to the total weight of the mixture or of the suspension, preferably from 0.5% to 1.5%; and/or
- the ratio % by weight of wax relative to the % by weight of oil is between 1:3 and 1:15, preferably between 1:5 and 1:12, the % by weight being expressed as a function of the total weight mixture or suspension; and/or
- the particle size reduction step is carried out by high-pressure homogenization or by emulsification by membranes or by micro-fluids; and/or
- the method comprises the addition of one or more preservatives and/or one or more antioxidants, preferably from 0.1 to 5%, in one of the phases or in an additional phase, preferably in the aqueous phase, such as 1,2-octanediol and/or propanol and/or citric acid; and/or
- the pH of said suspension is adjusted from 6 to 9, if necessary adjusted using a pH adjuster; and/or
- the method comprises an additional drying step wherein the water is removed to obtain a dried suspension; this step can be carried out by drying the aqueous phase to obtain a powder, for example by spray drying or lyophilization using for example a sugar such as mannitol or maltodextrin as a drying adjuvant.

A particularly interesting combination of features of the method according to the invention, suitable in particular for retinol encapsulation, is characterized in that:

- the waxy lipid phase consists of substantially:

  - 2.5 to 4% by weight of wax; and

  - 0.5 to 1.5% by weight of phosphatidylcholine or hydrogenated phosphatidylserine (S3);

- the oily lipid phase consists of substantially:

  - 20 to 30% vegetable oil; and

  - 2 to 4% retinol,

- the aqueous phase substantially comprises:

  - 1 to 3% by weight of lauryl glucoside or decyl glucoside (S1); and

  - 0.3 to 1.0% by weight of sorbitan monostearate, sorbitan laurate or sorbitan isostearate (S2);

all the percentages by weight being expressed according to the total weight of the mixture or the suspension and the complement q.s. to 100% by weight being provided by the weight of water.

According to second aspect of the present invention there is provided an aqueous suspension of submicron structured lipid particles which can be obtained by the method according to the invention, as defined according to the first aspect disclosed above.

According to preferred and advantageous features of the suspension according to the invention:

- the encapsulated hydrophobic compound is a hydrophobic vitamin, in particular selected from retinol, tocopherol derivatives, coenzyme Q10, resveratrol, ferulic acid, ascorbic acid, lipoic acid or salicylic acid, and their derivatives; and/or
- the % by weight of encapsulated retinol relative to the % by weight of said suspension is from 1 to 5%, preferably from 3 to 5%; more preferably the % by weight of encapsulated retinol relative to the % by weight of said suspension is at least 2%; and/or
- the size of the particles is from 0.08 to 5 $\mu$m, preferably from 0.1 to 2 $\mu$m, more preferably from 0.1 to 1 $\mu$m; and/or
- the polydispersity rate is 0.8 to 1.5 with a main particle size of from 0.1 to 0.6 $\mu$m; and/or
- the particles are substantially spherical in structure and have a waxy shell; in addition, they comprise an oily core containing said hydrophobic compound; and/or
- the suspension is in a dried form (a powder), which is an advantageous form for formulating makeup products, for example.

According to a third object, the present invention provides a composition, in particular an anti-aging cosmetic composition, but which can also be a pharmaceutical composition, comprising as active ingredient an effective amount of a suspension, as recited above according to the second aspect of the invention, and a physiologically acceptable medium. Such a composition is preferably topical.

"Physiologically acceptable" means that the compositions are suitable for topical or transdermal use, in contact with mucous membranes, appendages (nails, hair and body hair), scalp and skin of mammals, particularly human, compositions which may be ingested, or injected into the skin, without risk of toxicity, incompatibility, instability, allergic response, and others. This "physiologically acceptable medium" forms what is commonly called the excipient of the composition.

According to the invention, by "topical treatment" or "topical use" is meant an application which is intended to act on the site where it is applied: skin, mucous membrane and/or appendages.

A composition according to the invention can be applied to the face, body, neckline, scalp, hair, eyelashes, body hair, in any form or vehicle known to those skilled in the art, in particular in the form of a solution, dispersion, emulsion, paste or powder.

In cosmetics in particular, applications can be proposed in the ranges of skin care for the face, body, hair and body hair and ranges of make-up treatments, in particular eyelashes and eyebrows.

Furthermore, the composition may be incorporated onto a non-woven or woven material, with natural or synthetic fibers, wool, or any material intended to come into contact with skin and that can be used in clothing, including tights and socks, shorty, day or night underwear, tissues, handkerchiefs or fabric to exert its cosmetic effect *via* the contact skin/textile and enable continuous topical delivery (cosmetic textiles).

The galenical formulations can enter in different product ranges for personal care and/or beauty products including skin care, cleaning, makeup, cleansing, sunscreen, artificial tanning, pre-shave, shaving or aftershave, moisturizer, humectant, emollient, conditioning, exfoliating, astringent, depilatories or antiperspirant, deodorant, etc.

According to preferred and advantageous features, the composition of the invention may also comprise one or more other active ingredients.

The Personal Care Products Council ("International cosmetic ingredient dictionary & handbook" published by the "Cosmetic, Toiletry, and Fragrance Association, Inc.", Washington, D.C.) describes a non-limited wide variety of cosmetic and pharmaceutical ingredients conventionally used in the skin care industry that can be used as additional ingredients in the compositions for the present invention, as long as they are physically and chemically compatible with the other ingredients of the composition and especially with the active ingredients of the present invention. Also, the nature of these additional ingredients should not unacceptably alter the benefits of the active ingredient of the invention. These additional ingredients can be synthetic or natural such as plants extracts or issued from a bio-fermentation process.

Further skin care actives that are particularly useful combined with the composition according to the invention can be found in the commercial literature of Sederma, Crodarom and Alban Muller, and on the website www.croda.fr. Commercially available actives widely used in cosmetic compositions can also be mentioned as examples: betain, glycerol, Actimoist Bio 2™ (Active organics), AquaCacteen™ (Mibelle AGCosmetics), Aquaphyline™ (Silab), AquaregulK™ (Solabia), Carciline™ (Greentech), Codiavelane™ (Biotech Marine), Dermaflux™ (Arch Chemicals, Inc), Hydra'-Flow™ (Sochibo), Hydromoist L™ (Symrise), RenovHyal™ (Soliance), Seamoss™ (Biotech Marine), Essenskin™ (Sederma), Moist 24™ (Sederma), Argireline™ (commercial name of the acetyl hexapeptide-3 from Lipotec), spilanthol or an Acmella oleracea extract known under the trade name Gatuline Expression™, a Boswellia serrata extract known under the trade name Boswellin™, Deepaline PVB™ (Seppic), Syn-AKE™ (Pentapharm), Ameliox™, Bioxilift™ (Silab), PhytoCell-

Tec™ Argan (Mibelle), Papilactyl D™ (Silab), Preventhelia™ (Lipotec), or one or more of the following active ingredients sold by Sederma : Subliskin™, Venuceane™, Moist 24™, Vegesome Moist 24™, Essenskin™, Juvinity™, Revidrat™, Resistem™, Chronodyn™, Kombuchka™, Chromocare™, Calmosensine™, Glycokin factor S™, Biobustyl™, Idealift™, Ceramide 2™, Ceramide A2™, Ceramide HO3™, Legance™, Intenslim™, Prodizia™, Beautifeye™, Pacifeel™, Zingerslim™, Meiritage™, Sebuless™, Apiscalp™, Rubistem™, Citystem™, Neonyca™, NG Insaponifiables de Beurre de Karité™, Majestem™, Hydronesis™, Poretect™, Amberstem™, Synchrolife™, Sylverfree™, Feminage™, Ameyezing™, BB-Biont™, or mixture thereof.

Among plant extracts (in the form of classical plant extracts or prepared by an *in vitro* process) can be used as additional actives, there may more particularly be mentioned extracts of Ivy, for example English Ivy *(Hedera helix)*, *Bupleurum chinensis, Bupleurum falcatum,* arnica *(Arnica montana* L.), rosemary *(Rosmarinus officinalis* N.), marigold *(Calendula officinalis),* sage *(Salvia officinalis* L.), ginseng *(Panax ginseng),* gingko biloba, oSt.-John's-Wort *(Hyperycum perforatum)*, butcher's- broom *(Ruscus aculeatus* L.), European meadowsweet *(Filipendula* ulmaria L.), big- flowered Jarva tea *(Orthosiphon stamincus* Benth.), artichoke *(Cynara scolymus),* algae *(Fucus vesiculosus),* birch *(Betula alba),* green tea, cola nuts *(Cola nipida)*, horse-chestnut, bamboo, *Centella asiatica,* heather, fucus, willow, mouse-ear, escine, cangzhu, *Chrysanthellum indicum,* plants of the *Armeniacea* genus, *Atractylodis platicodon, Sinnomenum, Pharbitidis, Flemingia,* Coleus as *C. forskohlii, C. blumei, C. esquirolii, C. scutellaroides, C. xanthantus* and *C. barbatus,* such as the extract of root of *Coleus barbatus, Ballote, Guioa, Davallia, Terminalia, Barringtonia, Trema, Antirobia, Cecropia, Argania, Dioscoreae* such as *Dioscorea opposita* or Mexican, extracts of *Ammi visnaga, Siegesbeckia,* in particular *Siegesbeckia orientalis,* plant extracts of the family of Ericaceae, in particular bilberry extracts *(Vaccinium angustifollium)* or *Arctostaphylos uva ursi, Aloe vera,* plant containing sterols (e.g., phytosterol), Manjistha (extracted from plants of the genus Rubia, particularly *Rubia cordifolia),* and Guggal (extracted from plants of the genus *Commiphora,* particularly *Commiphora mukul*), kola extract, chamomile, red clover extract, Piper methysticum extract (Kava Kava™ from Sederma), *Bacopa monieri* extract (Bacocalmine™ from Sederma) and sea whip extract, extracts of *Glycyrrhiza glabra,* mulberry, *melaleuca* (tea tree), *Larrea divaricata, Rabdosia rubescens, Euglena gracilis, Fibraurea recisa hirudinea, Chaparral sorghum,* sun flower, *Enantia chlorantha,* Mitracarpe of *Spermacocea* genus, *Buchu barosma,* Law *sonia inermis L., Adiantium capillus-veneris L., Chelidonium majus, Luffa cylindrica,* "Japanese Mandari" *(Citrus reticulata Blanco* var. unshiu), *Camelia sinensis, Imperata cylindrica, Glaucium Flavum, Cupressus sempervirens, Polygonatum multiflorum, Loveyly hemsleya, Sambucus nigra, Phaseolus lunatus, Centaurium, Macrocystis pyrifera, Turnera diffusa, Anemarrhena* asphodeloides, *Portulaca pilosa, Humulus lupulus,* Coffea arabica, Ilex paraguariensis, *Globularia cordifolia, Oxydendron arboretum, Albizzia julibrissin, Zingimber zerumbet* smith*, Astragalus membranaceus, Atractylodes macrocephalae, Plantago lanceolata, Leontopodium alpinum* (or edelweiss), *Mirabilis jalapa, Apium graveolens, Marrubium vulgare, Buddleja davidii* Franch, *Syringa vulgaris, Engelhardia chrysolepsis,* or orchids.

A composition according to the third aspect of the present invention may include peptide(s), including, without limitation, di-, tri-, tetra-, penta-and hexapeptides and their derivatives.

In the frame of the present invention, the term "peptide" refers here to peptides containing ten amino acids or less, their derivatives, isomers and complexes with other species such as a metal ion e.g., copper, zinc, manganese, magnesium, and others). The term "peptides" refers to both natural peptides and (bio)synthetic peptides. It also refers to compositions that contain peptides and which are found in nature, and/or are commercially available.

Suitable dipeptides for use herein include but are not limited to Carnosine (beta-AH), YR, VW, NF, DF, KT, KC, CK, KP, KK, TT, PA, PM or PP.

Suitable tripeptides for use herein include, but are not limited to RKR, HGG, GHK, GGH, GHG, GKH, KPK, KFK, KavaK, KβAK, KabuK, KacaK, KPK, K(P)HG (comprising a proline (P) grafted on the lysine, KMOK, KMO$_2$K (MO$_2$ being a di-oxygenated sulfoxide methionine), PPL, PPR, SPR, QPA, LPA or SPA.

Suitable non limitative examples of tetrapeptides are GQPR (SEQ ID NO: 1), RSRK (SEQ ID NO: 2), KTFK (SEQ ID NO: 3), KTAK (SEQ ID NO: 4), KAYK (SEQ ID NO: 5), KFYK (SEQ ID NO: 6) or TKPR (SEQ ID NO: 7).

Suitable non limitative examples of pentapeptides are KTTKS (SEQ ID NO: 8) and KTSKS (SEQ ID NO: 9) and examples of hexapeptides are GKTTKS (SEQ ID NO: 10) and VGVAPG (SEQ ID NO: 11).

Other suitable peptides for use according to the present invention can be selected, this list being not limitative, from: lipophilic derivatives of peptides, preferably palmitoyl (Pal) derivatives or myristoyl (Myr), and metal complexes as aforementioned (e.g., copper complex of the tripeptide HGG or GHK).

Preferred dipeptides include for example N-Palmitoyl-beta-Ala-His, Pal-KT, Pal-RT or Pal-PP (Sederma).

Preferred tripeptide derivatives include for example the copper derivative of HGG (Lamin™ from Sigma), Pal-GKH and Pal-GHK (from Sederma), Lipospondin (N-Elaidoyl-KFK) and its analogs of conservative substitution, N-Acetyl-RKR-NH$_2$ (Peptide CK+), N-Biot-GHK (from Sederma), Pal-KAvaK, Pal-KβAlaK, Pal-KAbuK, Pal-KAcaK, or Pal-KMO$_2$K (Matrixyl®synthe'6® from Sederma), Pal-KVK (Syn-Coll™ of DSM), and derivatives thereof.

Mention may also be made here of the anti-aging tripeptides of general formula X-Pro*-Pro*-Xaa-Y described in WO2015181688, Xaa being selected from Leu, Arg, Lys, Ala, Ser, and Asp, at the N-terminus , X chosen from H, -CO-R$^1$ and -SO$_2$-R$^1$ and at the C-terminal end Y chosen from OH, OR$^1$, NH$_2$, NHR$^1$ or NR$^1$R$^2$, R$^1$ and R$^2$ being, independently of

one another, chosen from a alkyl, aryl, aralkyl, alkylaryl, alkoxy and aryloxy group, which may be linear, branched, cyclic, polycyclic, unsaturated, hydroxylated, carbonylated, phosphorylated and/or sulfurized, said group possibly possessing in its backbone a heteroatom particularly O, S and/or or N, and Pro* corresponding to Proline, an analogue or derivative thereof; comprising, for example, Myr-PPL-OH and Myr-PPR-OH.

Here can further be cited the propigmenting and/or pro-collagen dipeptides and tripeptides of general Formula X-(Xaa1)n-Pro*-Xaa2-Y disclosed in WO2014/080376, with n=0, 1 or 2, Xaa1 an hydrophobic aminoacid selected from Ala, Val, Met, Leu, Iso, Phe, Pro, and analogs and derivatives thereof; or a polar aminoacid selected from Ser, Thr, Tyr, Asp, Glu and analogs and derivatives thereof; and when n=2 the two aminoacids Xaa1 being the same or different; Xaa2 being an hydrophobic aminoacid selected from Ala, Val, Met, Leu, Iso, Phe, and analogs and derivatives thereof, or a basic aminoacid selected from Arg, Lys, His, and analogs and derivatives thereof; at the N terminal end X being selected from H, -CO-$R^1$ and -SO$_2$-$R^1$; at the C terminal end Y being selected from OH, O$R^1$, NH$_2$, NH$R^1$ or N$R^1$$R^2$; $R^1$ and $R^2$ being, independently from each other, selected from an alkyl, aryl, aralkyl, alkylaryl, alkoxy et aryloxy group, that can be linear, branched, cyclic polycyclic, saturated, unsaturated, hydroxylated, carbonylated, phosphorylated and/or sulphured, said group having or not an O, S and/or N heteroatom in its skeleton and Pro* corresponding to a Proline, analog or derivative thereof; comprising for example the following peptides Pal-SPR-OH, Pal-PPR-OH, Pal-QPA-OH, Pal-LPA-OH, Myr-SPA-OH, Pal-PM-OH, Pal-PA-OH and Pal-PP-OH.

Suitable tetrapeptides derivatives for use according to the present invention include, but are not limited to, Pal-KTFK (SEQ ID NO: 12), Ela-KTAK (SEQ ID NO: 13), Ela-KAYK (SEQ ID NO: 14), Ela-KFYK (SEQ ID NO: 15) or Pal-GQPR (SEQ ID NO: 16).

Suitable pentapeptides derivatives herein include, but are not limited to, Pal-KTTKS (SEQ ID NO: 17) (MATRIXYL™, Sederma), Pal-KTSKS (SEQ ID NO: 18) (BB-Biont™, Sederma), Pal-YGGFXaa (SEQ ID NO: 19) with Xaa being Trp, Phe, Tyr, Tic, 7-hydroxy-Tic or Tpi, or mixtures thereof.

Suitable hexapeptides derivatives for use herein include, but are not limited to, Pal-HLDIIXaa (SEQ ID NO: 20) with Xaa being Trp, Phe, Tyr, Tic, 7-hydroxy-Tic or Tpi, Pal-GKTTKS (SEQ ID NO: 21), Pal-VGVAPG (SEQ ID NO: 22) (DERMAXYL™, Sederma), or mixtures thereof.

The preferred compositions available commercially and sold by Sederma:

- tripeptides or a derivative thereof include Biopeptide-CL™, Maxilip™, or Procapil™ containing the peptide GHK, MATRIXYL™ Morphomics™ comprising Pal-K(P)HG;
- tetrapeptides or a derivative thereof include RIGIN™, Eyeliss™ containing Pal-GQPR (SEQ ID NO: 16) and an excipient, Crystalide™ containing Pal-KTFK (SEQ ID NO: 12) (solvated in a microemulsion);
- pentapeptide or a derivative thereif as Matrixyl™ containing Pal-KTTKS (SEQ ID NO: 17) or BB-Biont™ containing Pal-KTSKS (SEQ ID NO:18).

The two following mixtures of peptides can also be mentioned:

- the mixture of Pal-GHK and Pal-GQPR (SEQ ID NO: 16) (Matrixyl™ 3000), and
- the mixture of Pal-GHK and Pal-VGVAPG (SEQ ID NO: 22) (Biobustyl™).

The following marketed peptides can be mentioned as well as additional active ingredients:

- Vialox™ (INCI name = Pentapeptide-3 (synthetic peptide comprising alanine, arginine, isoleucine, glycine and proline)), Syn-ake™ (β-Ala-Pro-Dab-NH-Bzl) or Syn-Coll™ (Pal-Lys-Val-Lys-OH) marketed by Pentapharm;
- Argireline™ (Ac-Glu-Glu-Met-Gln-Arg-Arg-NH2 (INCI name = Acetyl hexapeptide-3) (SEQ ID NO: 23), Leuphasyl™ (Tyr-D-Ala-Gly-Phe-Leu) (SEQ ID NO: 24), Aldenine™ (Gly-His-Lys), Trylagen™ (INCI name = *Pseudoalteromonas* Ferment Extract, Hydrolysed Wheat Protein, Hydrolysed Soy Protein, Tripeptide-10 Citrulline (reaction product of Citrulline and Tripeptide-10 (synthetic peptide constituted of aspartic acid, isoleucine and lysine)), Tripeptide-1), Eyeseryl™ (Ac-β-Ala-His-Ser-His)(SEQ ID NO: 25), Serilesine™ (Ser-Ile-Lys-Val-Ala-Val) (SEQ ID NO: 26) or Decorinyl™ (INCI name: Tripeptide-10 Citrulline = reaction product of Citrulline and Tripeptide-10 (synthetic peptide constituted of aspartic acid, isoleucine and lysine) marketed by Lipotec;
- Collaxyl™ (Gly-Pro-Gln-Gly-Pro-Gln (SEQ ID NO: 27)) or Quintescine™ (Cys-Gly) marketed by Vincience;
- Cytokinol™LS (casein hydrolysate) marketed by Les Laboratoires Serobiologiques/Cognis;
- Kollaren™ (Gly-His-Lys), IP2000™ (Pal-Val-Tyr-Val) or Meliprene™ (INCI name = Monofluoroheptapeptide-1: reaction product of acetic acid and a synthetic peptide comprising arginine, glycine, glutamic acid, histidine, norleucine, p-fluorophenylalanine and tryptophan) marketed by l'Institut Européen de Biologie Cellulaire;
- Neutrazen™ (Pal-His-D-Phe-Arg-NH2) marketed by Innovations; or
- BONT-L-Peptide™ (INCI name = Palmitoyl Hexapeptide-19: reaction product of palmitic acid and Hexapeptide-19 (synthetic peptide constituted of asparagine, aspartic acid, lysine and methionine), Timp-Peptide™ (INCI name = Acetyl Hexapeptide-20: reaction product obtained by acetylation of Hexapeptide-20 (synthetic peptide constituted of alanine, glycine, lysine, valine and proline) or ECM Moduline™ (INCI name = Palmitoyl Tripeptide-28: reaction product of palmitic acid and Tripeptide-28 (synthetic peptide constituted of arginine, lysine and phenylalanine) marketed by Infinitec Activos.

It is also possible to envisage combining the composition of the invention with one or more cyclic peptides, in particular those extracted from linseed oil described in the Applicant's patent application FR1850845.

Preferably, according to the invention, the additional active ingredient is selected from vitamin B3, niacinamide, tocopherol, hexamidine, $\alpha$-lipoic acid, resveratrol or DHEA, hyaluronic acid, peptides, in particular N-acetyl-Tyr-Arg-O-hexadecyl, Pal-VGVAPG (SEQ ID NO: 22), Pal-KTTKS (SEQ ID NO: 17), Pal-KTSKS (SEQ IS NO: 18), Pal-GHK, Pal-KMO$_2$K, Pal-GQPR (SEQ ID NO: 16), Pal-KTFK (SEQ ID NO: 12) (Crystalide™, Sederma) and Pal-K(P)HG (MATRIXYL™ Morphomics™, Sederma) that are classic active agents used in topical cosmetic or dermo-pharmaceutical compositions.

According to a fourth aspect, the present invention provides the use of a suspension according to the invention as recited above according to the second aspect, or of a composition according to the invention as recited according to the third aspect, for a non-therapeutic cosmetic treatment of the skin and/or its appendages, orally or topically. The present invention thus covers a non-therapeutic cosmetic treatment method for beautifying or improving the appearance and general condition of the skin and/or appendages and treating their imperfections, comprising applying to a subject in need thereof an effective amount of the suspension according to the invention or of a composition comprising it in a physiologically acceptable excipient.

The effective amount according to the invention, namely its dosage in the composition, depends on the destination of the composition. It depends on various factors, such as the age, the condition of the patient, the severity of the disorder or disease and the administration mode. An effective amount means a non-toxic amount enough to achieve the desired effect.

These purely cosmetic treatments are in particular anti-aging treatments, to prevent or treat the effects of photo-ageing, in particular wrinkles and fine lines, unsightly spots, signs of skin fatigue such as dark circles and undereye bags, as it is with retinol treatments.

The suspension or the composition according to the invention can be applied locally to targeted areas.

For indication, for a cosmetic face treatment, the European Directive on Cosmetics has fixed a standard dosage of a cream at 2.72 mg/cm$^2$/day/person, and for a cosmetic body lotion at 0.5 mg/cm$^2$/day/person.

A cosmetic treatment method according to the invention can be combined with one or more other treatment methods targeting the skin such as luminotherapy, heat or aromatherapy treatments.

According to the invention, devices with several compartments or kits may be proposed to implement a method of treatment according to the invention which may include for example and non-restrictively, a first compartment containing a suspension according to the invention, and in a second compartment a complementary active ingredient, the compositions contained in the said first and second compartments in this case being considered to be a combination composition for simultaneous, separate or stepwise use in time, particularly in one of the treatment methods recited above.

According to a fifth aspect, the present invention provides a suspension according to the invention as recited above according to the second object, or a composition according to the invention as recited according to the third object, for use in a therapeutic treatment, preferably topically.

According to a sixth aspect, the present invention provides the use of a suspension according to the invention as recited above according to the second object, as an active ingredient, for the manufacture of a physiologically acceptable composition suitable for the treatment of the skin and/or its appendages, orally or topically.

DETAILED DESCRIPTION

The present invention will be better understood in the light of the detailed description of embodiments and the studies described below.

Description of the Figures:

Figure 1 schematically illustrates the morphology of the particles according to the invention.
Figure 2 is a curve illustrating the particle size distribution.
Figure 3 is an image obtained by scanning electron microscopy (SEM).
Figure 4 is an image obtained by transmission electron microscopy (TEM).
Figure 5 is a graph of thermogravimetric analysis (TGA) of free retinol.
Figure 6 is a graph of TGA analysis of a simple emulsion.
Figure 7 is a TGA analysis graph of a suspension according to the invention.
Figure 8 is a TGA analysis graph of a placebo suspension.
Figure 9 is a differential scanning calorimetric analysis graph (DSC) of a suspension according to the invention.

Figure 10 is a DSC graph of free retinol.

Figure 11 is a DSC graph of cetyl palmitate alone.

Figure 12 is a graph of the evolution over time of the particle size of a suspension according to the invention to illustrate the stability of the suspension.

Figure 13 is a diagram of a Franz cell.

Figure 14 is a graph illustrating the release profile of free retinol compared to encapsulated retinol according to the invention.

Figure 15 is a chromatogram of free retinol at t=10h.

Figure 16 is a chromatogram of free retinol at t=48h.

Figure 17 is a chromatogram of the suspension according to the invention at t=24h.

Figure 18 is a chromatogram of the suspension according to the invention at t=48h.

Figure 19 is an optical microscope image (HE staining) on which is superimposed the Raman spectrum of a section of skin explant without product applied (control) at t=8h.

Figure 20 is a figure similar to Figure 19 at t=24h.

Figure 21 is an optical microscope image (HE staining) on which is superimposed the Raman spectrum of a section of skin explant to which free retinol was applied at t=8h.

Figure 22 is a figure similar to Figure 21 at t=24h.

Figure 23 is an optical microscope image (HE staining) on which is superimposed the Raman spectrum of a section of skin explant to which encapsulated retinol according to the invention has been applied at t=8 h.

Figure 24 is an optical microscope image (HE staining) on which is superimposed the Raman spectrum of a section of skin explant to which encapsulated retinol according to the invention has been applied at t=24 h.

EXAMPLE 1:

1. Manufacturing of a suspension of retinol particles according to the invention

Raw materials (all weight % are expressed relative to the total weight of the mixture formed by the different phases):

Waxy phase:

Wax: Cetyl Palmitate: 2.5 to 4%
S3: Hydrogenated lecithin: 0.5 to 1.5%

Oily phase:

Oil: Soybean oil: 20 to 30%
Hydrophobic molecule: retinol: 2.0 to 4%

Aqueous phase:

S1: Lauryl glucoside: 1 to 3% (HLB between 12 and 14)
S2: Sorbitan stearate: 0,3 to 1 % (HLB = 4,8)
Water: qs 100 %

Optionally: 0.1 to 5% by weight of one or more preservatives, for example 1,2-octanediol and/or propanol, and/or one or more antioxidants (for example acid citric) in one of the phases or in an additional phase.

The wax/oil ratio is between 1/5 and 1/12.

Protocol:

Step 1: preparation of the aqueous phase: water + S1 + S2, heating to dissolve the components.

Step 2: preparation of the waxy phase: wax + S3, heating to dissolve the wax and the lecithin.

Step 3: preparation of the oily phase: oil + retinol.

Step 4: hot mixing of the three phases: aqueous, wax and oil, each phase being substantially liquid and homogeneous.

Step 5: formation of a pre-emulsion.

Step 6: Particle size reduction by homogenization under hot high pressure.

Step 7: cooling to room temperature to form the suspension of submicron capsules according to the invention.

2. <u>Characterisation:</u>

2.1. Macroscopic characterisation:

A homogeneous suspension is obtained, with no visible phase separation, of a very pale-yellow milky colour and no particles are visible. The formulation has a low viscosity. It is miscible with water.

The pH of the suspension is 8.2 $\pm$ 0.1.

The average density of the suspension is 0.98 $\pm$ 0.01 g/ml.

The average retinol content of the samples just after preparation is 3.58% $\pm$ 0.04 m/v.

(Retinol content was quantified using an HPLC method with a C18 column, water:methanol (5:95 v/v) mobile phase, 35°C temperature, 1.0 ml/min flow rate, 20 $\mu$l injection volume and 8 min run time, and a UV-Vis detector at 325 nm. Samples were diluted in isopropanol. Final sample concentration was 2.5 mg/ ml. A calibration curve was performed.

2.2. Characterisation of the particles:

Size:

The laser diffraction technique (Mastersizer 3000™, Malvern). The suspension was added directly to the wet disperser unit containing water until darkening in the 2-8% range.

Analysis showed that the suspension had a refractive index of 1.456.

The particle size distribution curve is presented in Figure 2. The results are expressed in $D_V(50)$ and $D_V(90)$, corresponding respectively to the particle diameters at the 50th and 90th percentiles of the distribution curve.

Figure 2 shows that the particle size is homogeneous with a low polydispersity: the median $D_V(50)$ is 0.28 $\pm$ 0.02 $\mu$m and the $D_V(90)$ is 0.46 $\pm$ 0.01 $\mu$m. The span is the value that shows the polydispersity. It is calculated as $(d_{90}-d_{10})/d_{50}$. The smaller the range is, the narrower the polydispersity is.

The span averaged 1.04 $\pm$ 0.09, indicating a narrow distribution. In the literature, a span less than 2 is considered as a narrow distribution.

Zeta potential and particle size by dynamic light scattering (DLS):
The zeta potential was measured using a Zetasizer™ equipment. Samples of suspension at 105 days of production were diluted 2,000x in water, then stored at room temperature (RT), 40°C and 50°C for 90 days, then 15 days at room temperature.

A negative zeta potential was measured for all samples.

The sample stored at RT exhibited a zeta potential of -59.7 mV.

The sample stored at 40°C exhibited a zeta potential of -61.3 mV.

The sample stored at 50°C showed lower retinol content and reduced zeta potential to -37.5 mV.

Particle size was also evaluated for these samples. They exhibited an average diameter of 232, 223 and 217 nm for storage at RT, 40°C and 50°C. Polydispersity values ranged from 0.143 to 0.196, all considered very low correlated with a narrow size distribution.

Particle morphology:

Analysis by scanning electron microscopy (SEM):

Objects in relief can be seen by SEM. Images like the one in Figure 3 were taken by a SEM FEG apparatus at 20 kV accelerating voltage and an 80,000 magnification.

Thanks to this imaging technique, and as illustrated in Figure 3, it was possible to verify that the particles are substantially spherical, with a rough surface, and their size is compatible with the analysis by laser diffraction (presented above).

Analysis by transmission electron microscopy (TEM):

The suspension diluted at 5% in water was applied to a support and dried for 1 min. The sample was then stained with 2% uranyl acetate for 1 min.

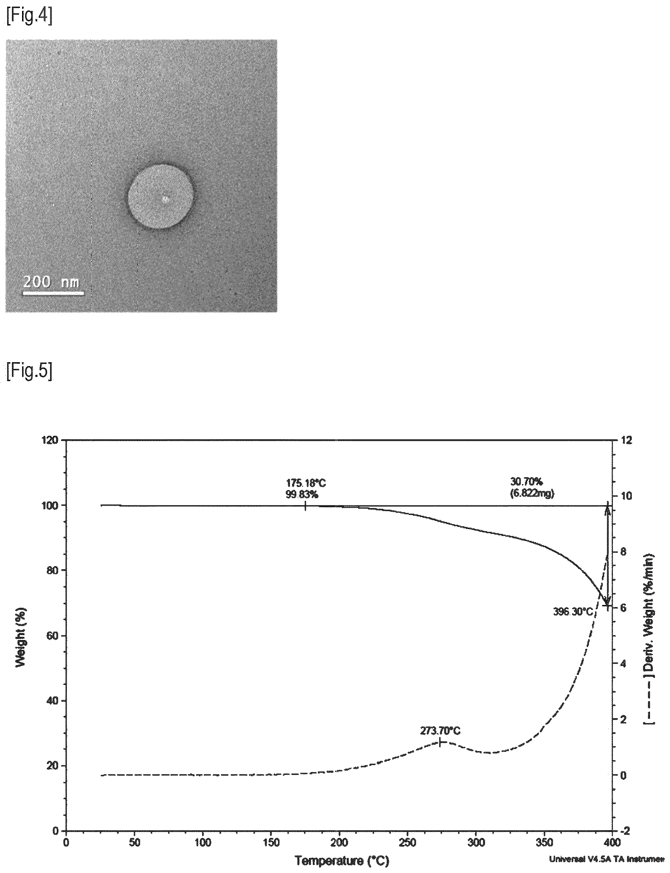

Thanks to this technique and as illustrated in Figure 4, it was possible to verify that the particles according to the invention are substantially spherical. The lauryl glucoside coating is visible around the sphere. The particle size is also compatible with the laser diffraction analysis (presented above).

Thermal analysis

Two analyses were carried out:

- a differential scanning calorimetric analysis (DSC) at 10°C/min from -20 to 180°C.
- a thermogravimetric analysis (TGA) carried out under a nitrogen atmosphere and at 10°C/min from ambient temperature to 400°C.

Samples tested:

- Free retinol (10% w/w retinol in soybean oil)
- Emulsion made from the same formula as Example 1 without the wax
- Suspension according to the invention comprising the particles of encapsulated retinol
- Placebo suspension according to the invention comprising the particles without retinol

Figures 5 to 8 correspond respectively to the graphs of the TGA analysis of free retinol, the simple emulsion, the suspension according to the invention and the placebo suspension.

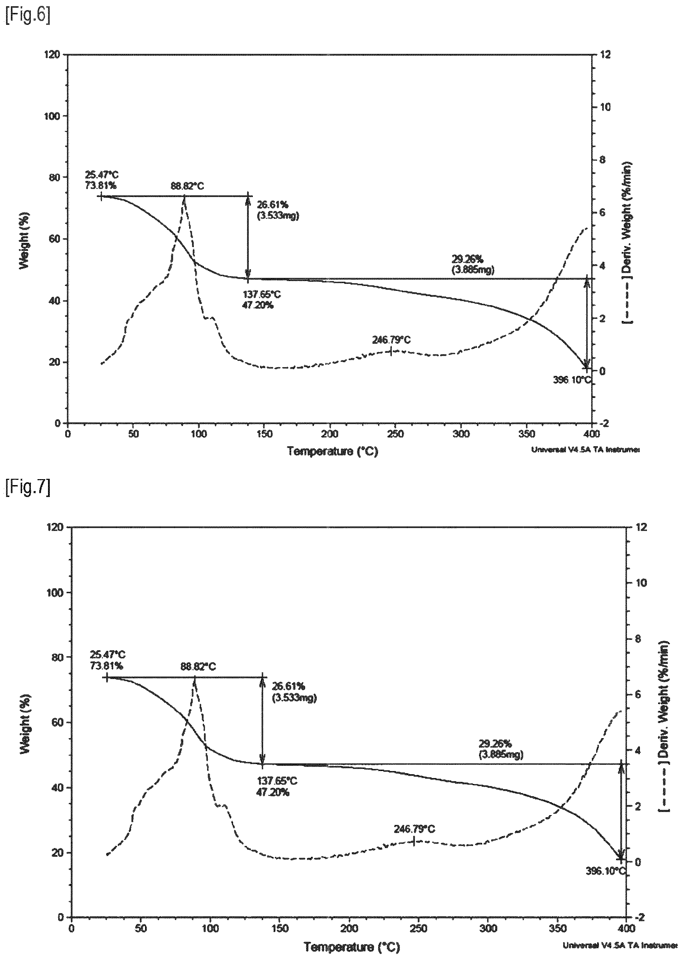

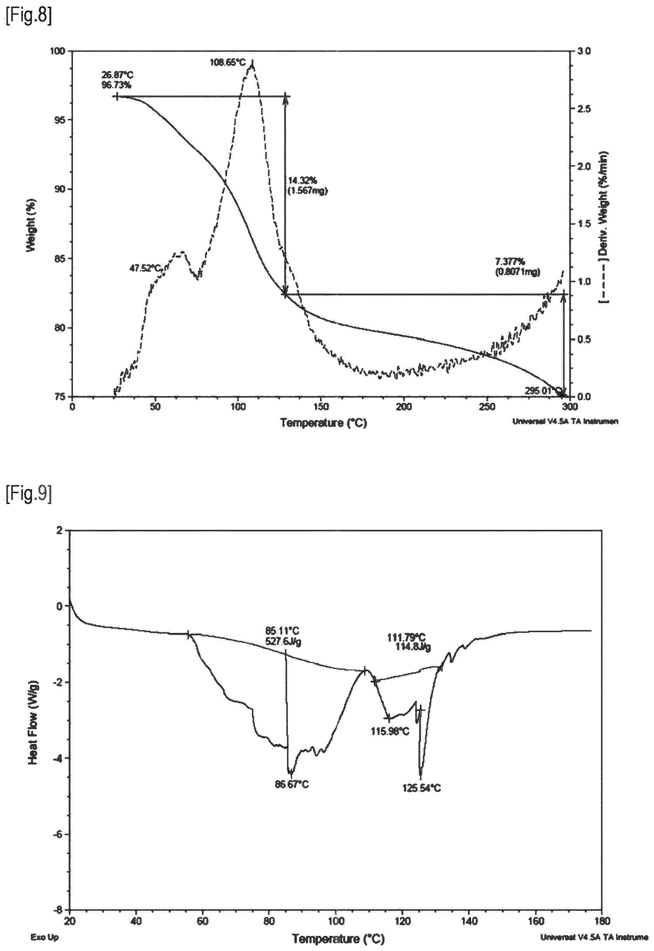

- The free retinol graph (Figure 5) shows that the retinol peak appears between 243-278°C.
- On the graph of the emulsion (Figure 6) the retinol peak is found again as well as another peak corresponding to the oily core at 81.6°C
- In comparison on the graph of the emulsion according to the invention (Figure 7), there are two peaks of the wax mixture of the shell at 42.4°C and 55.98°C, separated from the peak corresponding to the oil at 81.6°C.
- The graph of the placebo suspension (Figure 8) confirms the peaks corresponding to the shell and the core.

Figures 9 to 12 correspond respectively to the DSC thermograms of free retinol, the simple emulsion, the suspension according to the invention and the placebo suspension.

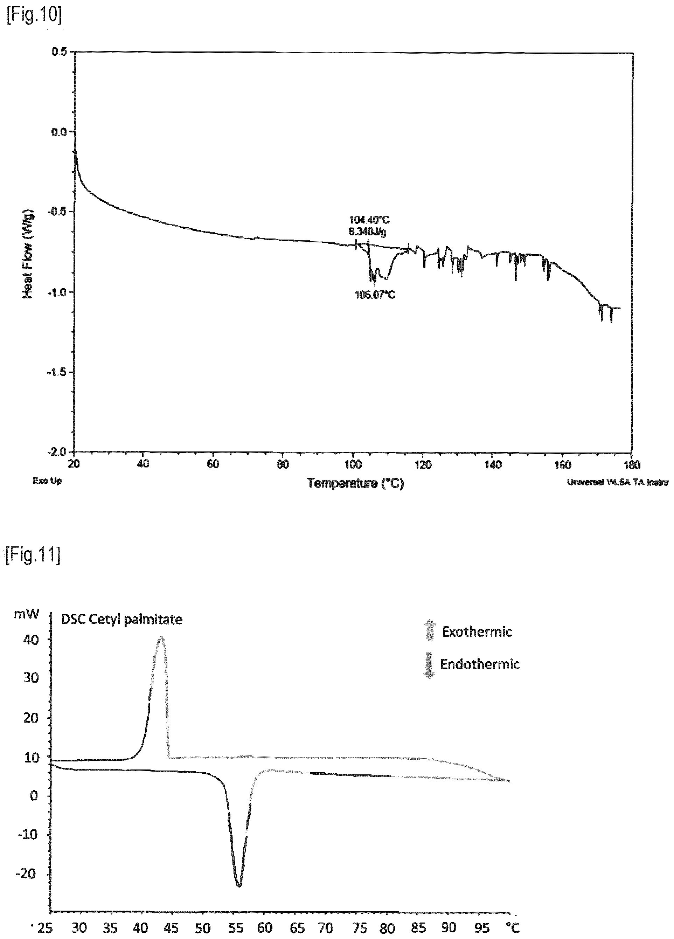

Figure 10 shows the thermogram of free retinol (solubilized in oil). It shows a retinol melting peak at 104-106°C.

- On the thermogram of the placebo suspension (Figure 11) it can be seen that the wax (cetyl palmitate) has a single well-defined melting peak at 54°C.
- On the thermogram of the free retinol (Figure 9) it can be seen that the free retinol dissolved in the soybean oil showed two endothermic peaks at 104 and 106°C.
- The thermogram of the suspension according to the invention (Figure 11) showed a large endothermic peak at 86°C corresponding to the melting peaks of the wax (cetyl palmitate), the second surfactant (Span 60) and the third surfactant (lecithin).

Conclusion on the particle morphology:

SEM images showed round spherical particles with a rough surface. TEM images showed a definite shell and structure, supporting the spherical shape.
TGA analysis showed the presence of crystalline components of the waxy shell.
DSC analysis showed a large combined endothermic peak attributed to the mixture of wax and surfactants.
Based on these results, it is possible to conclude that the particles of the suspension according to the invention are structured at ambient temperature as shown in Figure 1, with a solid shell and a liquid core with a very high oil content, such a structure being characteristic of that of capsules.

3. Evidence of encapsulation:

Fluorescence microscopy was used to prove encapsulation. The fluorescent nature of retinol was visualized by a blue colour demonstrating its presence.

Tested samples:

- A free retinol formulation (soybean oil + 10% w/w retinol).
- A particle suspension according to the invention.

- A suspension of placebo particles, the particles containing soybean oil but no retinol.
- A suspension of placebo particles containing free retinol (soybean oil + 10% w/w retinol).

Protocol:

Samples were visualized in both visible light and fluorescence (372 nm excitation and 456 nm emission wavelengths).

Optical microscopy was used as well as electron microscopy for more detailed images of the particles.

In optical microscopy, under visible light, free retinol was seen as a homogeneous dark, particle-free, blue image with fluorescence.

In the placebo suspension doped with free retinol, the droplets of free retinol are easily visible under the optical microscope, due to their larger size.

The suspension with the placebo capsules was seen under fluorescence as a black image, different from the blue of the free retinol. When retinol is encapsulated, retinol fluorescence is reduced, and the image is dark blue. When the placebo sample enriched with free retinol was observed, large blue droplets could be observed demonstrating fluorescence of unencapsulated retinol.

No visible quantity of free retinol is observed in the microscopies of the suspension of particles according to the invention.

Based on these observations, it was possible to conclude that the retinol is actually encapsulated in the particles of the suspension according to the invention, and that the size of the particles is reduced and homogeneous.

4. Encapsulation efficiency:

Encapsulation efficiency was evaluated by the ultrafiltration/centrifugation method using the following equation:

$$EE = ((C_{total} - C_{free})/C_{total}) \times 100$$

$C_{total}$ is the total amount of retinol in the sample (3.58% $\pm$ 0.04 m/v). $C_{Free}$ is the amount of unencapsulated retinol found in the external phase. To measure $C_{free}$, a suspension sample according to the invention (2 ml) was placed in a centrifuge (Vivaspin turbo 4™, PES 30 KDa membrane). The tube was centrifuged at 4000 rpm for 20 min. The external phase which was filtered was collected and the retinol content was evaluated by HPLC.

To ensure that retinol does not adhere to the membrane, an emulsion of water, 2.5% lauryl glucoside and 27% soybean oil and 3% retinol was prepared. The emulsion was centrifuged, and the quantity of retinol recovered after centrifugation was measured, and actually corresponds to the initial quantity.

The EE was calculated to be 100%, demonstrating that the total amount of retinol added to the formulation was encapsulated in the particles.

5. Stability:

5.1. Evolution of the retinol content:

For an accelerated stability study, suspension samples according to the invention were stored for 365 days at room temperature (RT), 180 days at 40°C, 90 days at 5°C and 60 days at 50°C.

The long-term stability study was evaluated at RT. Samples were analysed for characterization parameters at test times 0, 7, 14, 21, 30, 60, and 90, 180 and 365 days, depending on each storage condition. At each analysis time, a new bottle was opened for the first time.

To assess the impact of product handling during stability, an additional bottle of each sample was stored at RT. This same bottle was opened and handled under an air atmosphere at each analysis time (7, 14, 21, 30, 60 and 90 days for all samples for RT) and the results were compared to the cases of the bottles newly opened for each analysis.

All samples were handled without direct contact with light and under normal air conditions. No inert gas was used either to fill the bottles or to handle any sample. The results are given in the table below.

Table 1:

| | Storage (days) | 0 | 7 | 14 | 21 | 30 | 60 | 90 | 180 | 365 |
|---|---|---|---|---|---|---|---|---|---|---|
| | | Retinol content in w/w % | | | | | | | | |
| New bottle for each test | RT | 3.58 | 3.44 | 3.52 | 3.58 | 3.46 | 3.28 | 3.16 | 3.17 | 2.46 |
| | 5°C | 3.58 | 3.19 | | | 3.23 | 2.83 | 2.89 | | |
| | 40°C | 3.58 | 3.05 | | | 2.95 | 2.67 | 2.34 | 2.20 | |
| | 50°C | 3.58 | 3.00 | 3.03 | 3.01 | 2.80 | 2.21 | | | |
| Reusing of the same bottle | RT | 3.58 | 3.18 | 3.16 | 3.17 | 3.09 | 2.53 | 2.48 | | |
| Free retinol | RT | 3.11 | 2.76 | 2.40 | 0.23 | 0.25 | | | | |
| | 5°C | 3.11 | 3.14 | 2.77 | 0.42 | 0.0 | | | | |
| | 40°C | 3.11 | 2.79 | 0.89 | 0.0 | 0.0 | | | | |
| | 50°C | 3.11 | 2.51 | 0.10 | 0.0 | 0.0 | | | | |

The results in Table 1 show that retinol, when it is encapsulated according to the method of the invention, has a much greater stability over time compared to free retinol. The stability of the encapsulated retinol according to the invention is maintained even at high temperatures of 40°C and 50°C. Unlike non-encapsulated retinol, the encapsulated retinol according to the invention is stable at RT and does not need to be stored under cold.

It is also seen that thanks to the encapsulation according to the invention, the manipulation can be done under an air atmosphere.

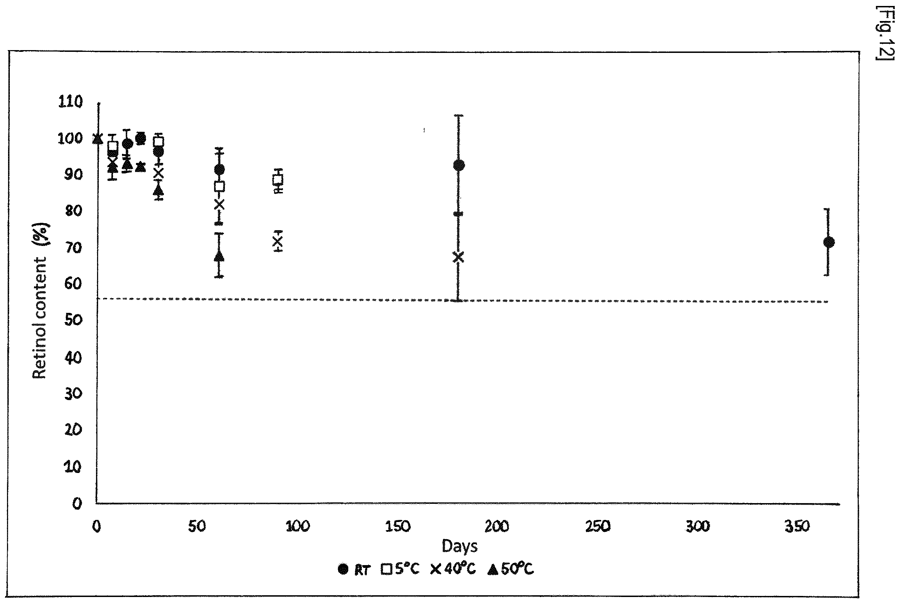

The decrease in retinol content compared to the initial concentration is visible in Figure 12. The trend is visible up to one year at RT. Advantageously, the level of retinol encapsulated according to the invention has been preserved at more than 2% for one year, corresponding to a concentration of 55% of that of the original concentration. The dotted line shows this minimum of 2% which was expected, and which was advantageously achieved thanks to the invention, all the values being above this limit.

5.2. Colour evolution:

No significant colour change occurred in the stored samples. Comparing samples at times 0, 30, 60 and 90 days, the colour change is very subtle, and all samples continue to be classified as very pale yellow.

5.3. pH evolution:

No significant change in pH occurred in the stored samples. The pH reduction was 1 point after 6 months at room temperature, which is characteristic of submicron lipid systems, as described in the literature.

5.4. Evolution of the particle size

A study over 90 days shows that the samples have good granulometric stability.

5.5. Study of UV stability of encapsulated retinol

Aim: to study whether the encapsulated retinol according to the invention supported UV irradiation and to compare with the non-encapsulated free retinol (solubilized in soybean oil).

Protocol: two samples, one containing free retinol and the other encapsulated retinol, were irradiated for 1 hour at about 40cm from the bulb of the solar lamp.

Retinol assay: by HPLC (UV detection at 325nm), the samples having been solubilized and diluted in isopropyl alcohol and the quantification having been carried out using a standard curve ranging from 2 to 150 ppm of retinol.

Results:

Table 2:

| | Actual content of retinol (in weight %) | | Degradation % of the retinol | |
|---|---|---|---|---|
| | Free retinol | Encapsulated retinol | Free retinol | Encapsulated retinol |
| Control | 10.49 +/- 0.34 | 3.14 +/- 0.01 | Ref. | Ref. |
| After 1h of irradiation | 5.72 +/- 0.07 | 2.50 +/- 0.04 | -45.38 +/- 2.2 | -20.36 +/- 1.34 |

The results show that encapsulated retinol is better protected after irradiation with a sunlamp than free retinol. It can be concluded that encapsulation using the method according to the invention protects the hydrophobic active ingredient, here retinol, from UV degradation.

6. Study of the retinol release profile:

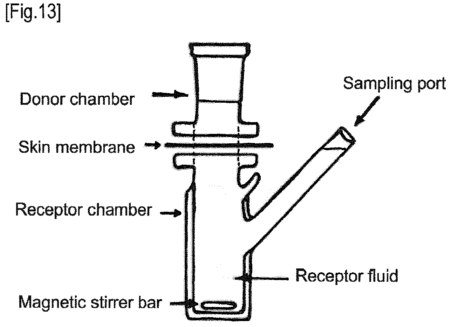

The Franz diffusion cell is the device most used to examine the release profiles of an active ingredient encapsulated in the capsules according to the invention. As seen in Figure 13, the cell is made up of two chambers, upper and lower, separated by a synthetic membrane. The product to be tested is placed in the upper chamber while the lower chamber collects the recipient fluid which has passed through the membrane.

The release medium is chosen to ensure optimal flow conditions, namely a concentration level of active that avoids diffusion interference.

The membrane is chosen to prevent the capsules from crossing the membrane intact, while the active must be able to cross it.

Rate and mechanism of the release of the active

The rate and mechanism of release can be obtained by mathematical modelling.

The rate can be calculated using the following first-order equation:

$$C = 100 - e^{-kt}$$

And the release mechanism can be obtained using the Korsmeyer-Peppas model equation:

$$M_t / M_\infty = k_{KP} \times t^n$$

In which:

- t is the release time
- $M_t$ is the quantity of released active at t time
- $M_\infty$ is the total quantity of released active
- $M_t/M_\infty$ being the fraction of released active at t time;
- k is a kinetic constant, a measure of the release rate; and
- n the diffusional exponent which gives an indication of the release mechanism of the active and takes on different values depending on the geometry of the release device.

Protocol:

Chosen release medium: 3% Tween 80 / 97% water (v/v).

Selected membrane: PVDF (polyvinylidene fluoride)) 0.1 $\mu$m.

Eight Franz cells containing the release medium and the membrane were prepared and placed in a temperature-controlled water bath of 32°C. Four cells were provided to evaluate the free retinol (retinol solubilized at 10% w/w in soybean oil) and four for the suspension according to the invention. The amount added to the membrane was 20% of the saturation concentration to ensure flow conditions.

Samples were taken at selected intervals of 0.5 to 76 h with replacement of fresh medium. All samples collected were analysed for retinol content by HPLC on the day of the experiment.

Results:

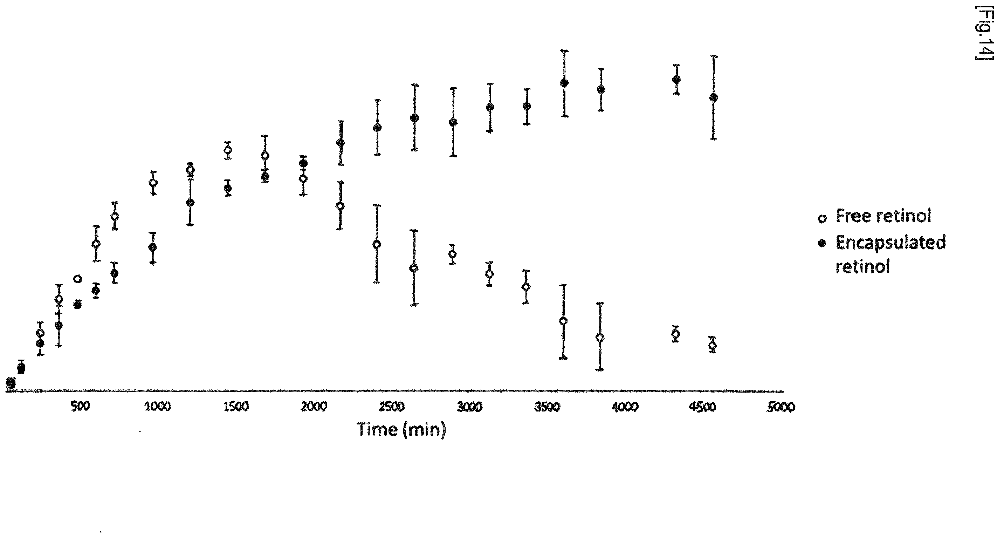

Figure 14 shows the release profile obtained from free retinol and encapsulated retinol. The mean of the four cells in both cases was plotted against time and used to apply the mathematical models. Using the models, the release rate was calculated as well as the release time $t^{1/2}$, which indicates the time required to release 50% of the retinol content. The release mechanism was obtained by the Korsmeyer-Peppas equation.

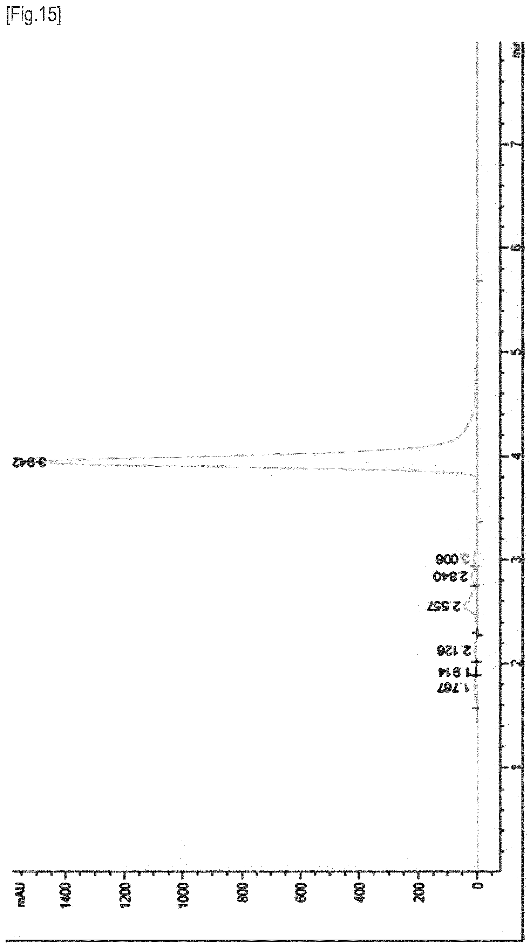

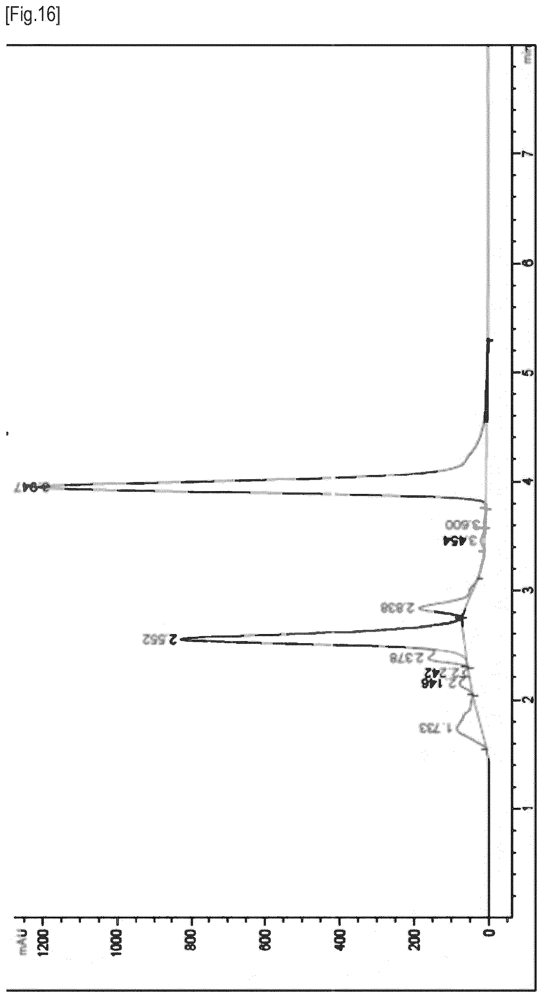

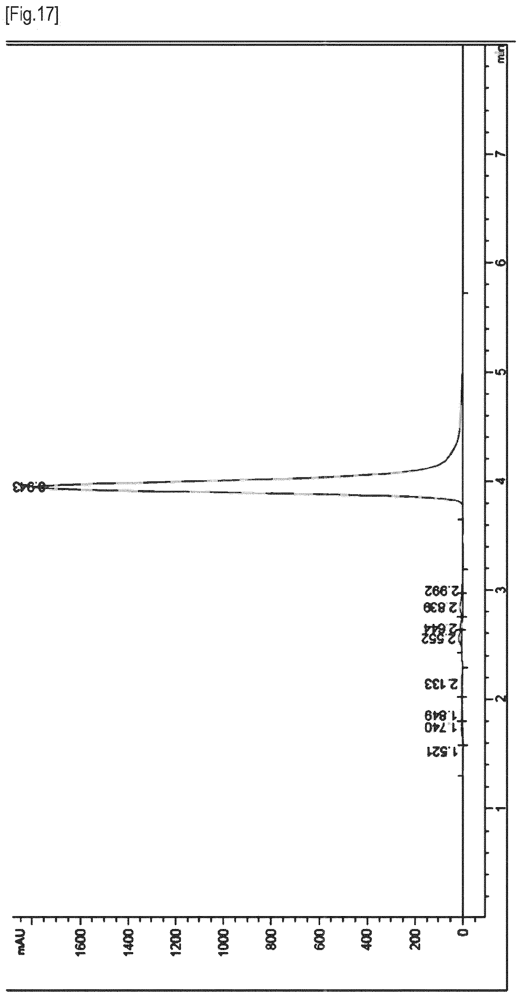

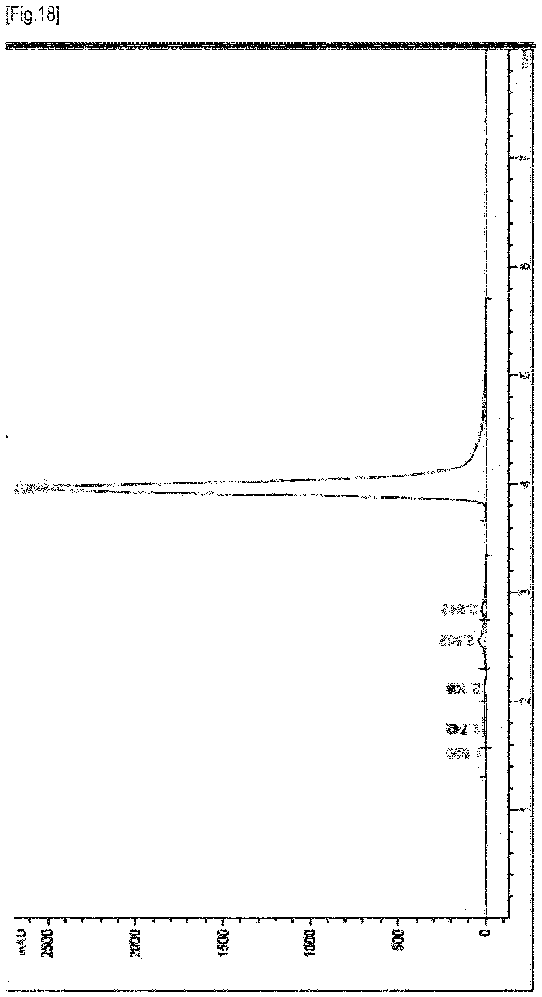

The curve show that the free retinol was released for up to 24 hours with an increased concentration. The chromatograms in Figure 15 and Figure 16 show degradation peaks from 10h. On its side, the encapsulated retinol suspension according to the invention released retinol in a slow and continuous process reaching a higher retinol concentration than that of the free sample. The chromatograms in Figure 17 and in Figure 18 indeed show retinol degradation peaks which are only visible after 24 hours and over a very small area compared to the samples of free retinol. It can be seen from the curve in Figure14 that the release of free retinol was faster than the release of encapsulated retinol according to the invention.

The retinol suspension according to the invention released retinol and followed an exponential release pattern. The release half-life was calculated to be 1074 min. The release rate was 0.00064 $min^{-1}$. The free retinol was released initially following the exponential curve and this up to 24 h. During the release phase, in the presence of small amounts of degraded retinol, the release rate was 0.00089 $min^{-1}$ and the release half-life was 772 min. After 24h, the degradation occurred in a more expressive way than the release rate, and a reduction in the concentration was observed in a $t^{1/2}$ of 122 min also following an exponential decrease.

The release mechanism was also calculated. The exponent n was calculated based on the Korsmeyer-Peppas equation at 0.69, which indicates abnormal non-Fickian transport, which is a combination of Fickian and non-Fickian processes and, in this case, a combination of diffusion and erosion process.

7. Skin penetration study

The study was performed on live human skin explants using Raman spectroscopy.

Explants used: abdominal skin of 12mm +/- 1mm

Products tested, applied to the explants:

- retinol encapsulated according to the invention: the cream formulation of the galenic part below but containing 9% w/w of suspension according to the invention, namely 0.27% w/w of retinol; and

- free retinol: the cream formulation identical to that used for encapsulated retinol but containing, instead of the suspension, 9% w/w of a mixture of soybean oil with a final concentration of 0.27% w/w of retinol.

After pre-processing the images, the spectral images were reconstructed using an adjustment procedure considering the reference spectra of the products and the control. Each pixel spectrum is represented by a linear combination of corresponding reference spectra. The reconstructed spectral images allow to see the level of penetration and the spatial distribution of each product in the skin after 8h and 24h of test time.

Results:

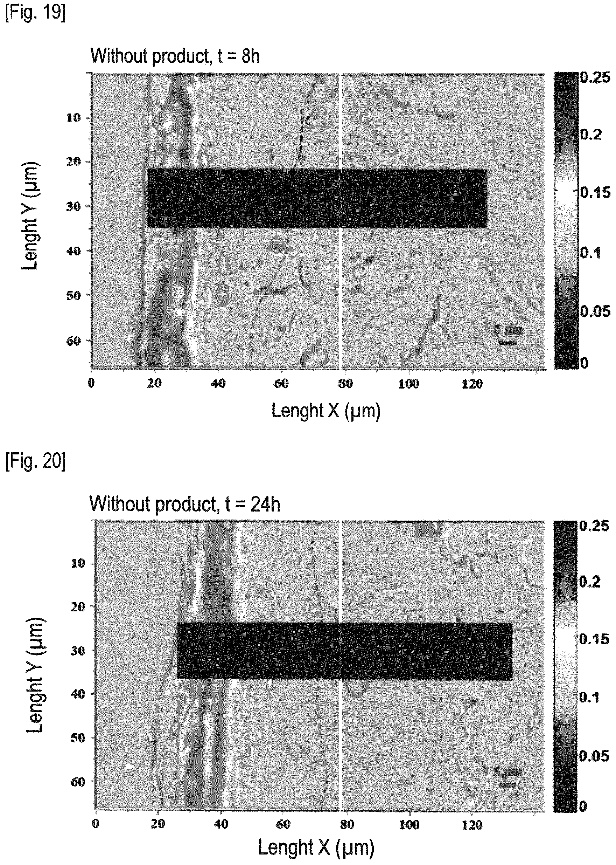

For the skin explants where no product was applied, the black spectral bands show that no signal corresponding to retinol was observed at 8h (Figure 19) and 24h (Figure 20).

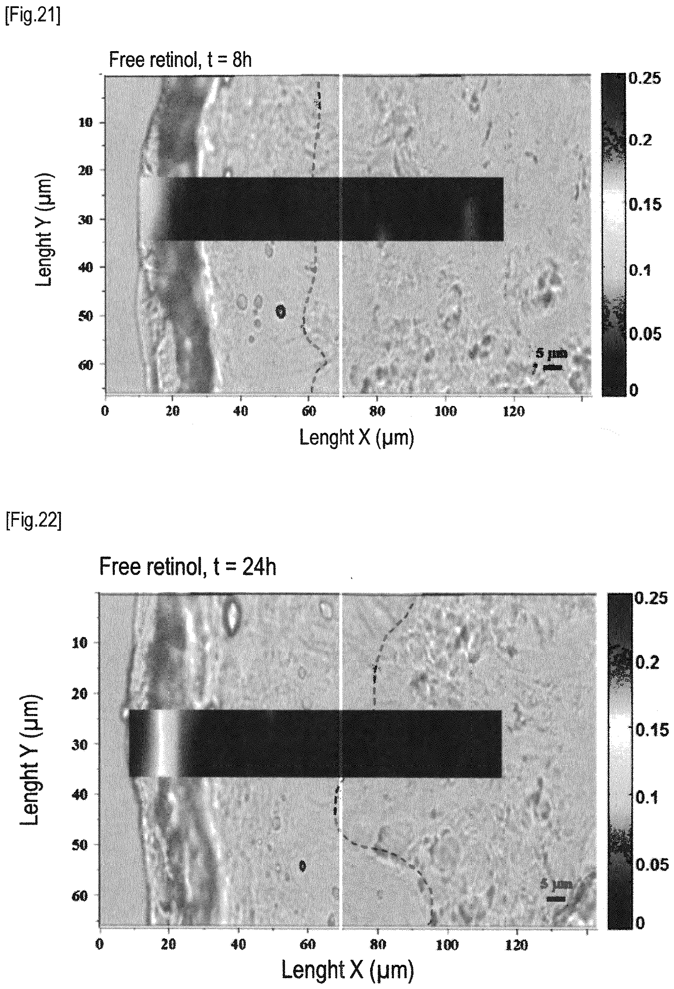

For the skin explants on which free retinol has been applied, it can be seen in Figure 21 that at 8h only a few signals are visible on the spectral band at the *stratum corneum* level, and it can be seen in Figure 22 that at 24h the retinol signals are all concentrated on the spectral band at the *stratum corneum* level, and that there is no signal at the level of the epidermis.

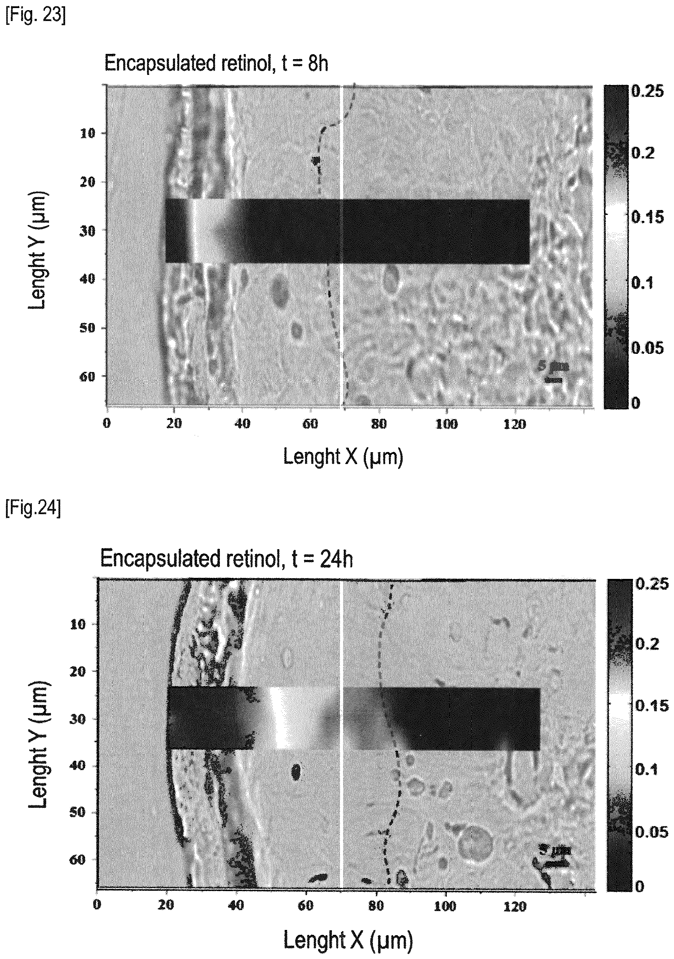

For encapsulated retinol, it can be seen in Figure 23 that after 8h, the retinol is concentrated throughout the *stratum corneum,* and in Figure 24 that after 24h, the retinol is not only present in the *stratum corneum* but also in the epidermis.

COMPARATIVE EXAMPLES

Choice of the surfactant system:

Table 3:

|  | S1 HLB>10 | S2 HLB<6 and solid at RT | S3 Phospholipid | Formation of a suspension of submicron particles at T0? | Stability over time |
|---|---|---|---|---|---|
| Invention (as Example 1) | X | X | X | Yes: suspension is homogeneous, stable, without phase separation, without visible particles | 1 year of stability (cf. results given above) |
| Comparative Example 1 | X |  |  | No: formation of a viscous product similar to a cream and not containing particles of submicronic size | / |
| Comparative Example 2 | X |  | X | Yes: suspension is homogeneous, stable, without phase separation, and without visible particles | Approximately 1 month, then phase separation occurs and a loss of more than 20% encapsulation |
| Comparative Example 3 | X | X |  | No: 2 heterogeneous phases, similar to a lotion | / |

Nature of the second surfactant S2:

Table 4:

|  | Formation of a suspension of submicrometric particles at T0? | Stability over time |
|---|---|---|
| Span 60 (solid at RT) | Yes | Yes |
| Span 80 (liquid at RT) | No. Particle size is greater than 30 microns. | / |

GALENICAL

Galenic formulations can be prepared, comprising as active ingredient a suitable percentage by weight of a suspension according to the invention encapsulating an active molecule, for example retinol, as prepared according to the example described above.

These formulations can contain additional cosmetic active agents, the latter coming, if necessary, in support and/or in addition to the activity of the active ingredient according to the invention. These ingredients can be of any category depending on their function(s), the place of application (body, face, neck, chest, hands, etc.), the desired final effect and the targeted consumer, for example anti-aging, moisturizing, firming, anti-redness, anti-stretch marks, sunscreen, etc.

Formulation example: cream form

Table 5:

| Raw materials | INCI name | Ingredient (w/w%) |
|---|---|---|
| Phase A |  |  |
| $H_2O$ | Water | qsp100 |
| Carbopol Ultrez 10 ™ | Carbomer | 0.30 |
| Phase B |  |  |
| Brij S2-SS-(RB)™ | Steareth-2 | 0.40 |
| Brij S10-SO-(RB)™ | Steareth-10 | 1.20 |
| Crodafos CES-PA-(RB) ™ | Cetearyl Alcohol (and) Dicetyl Phosphate (and) Ceteth-10 Phosphate | 4.00 |
| Crodacol CS90-PA-(RB)™ | Cetearyl Alcohol | 1.50 |

(continued)

| Phase B | | |
|---|---|---|
| Crodamol AB-LQ-(RB) ™ | C12-15 Alkyl Benzoate | 1.50 |
| Crodamol OSU-LQ-(JP) ™ | Diethylhexyl Succinate | 7.00 |
| Phase C | | |
| Glycerin | Glycerin | 2.50 |
| Octanediol | Caprylyl Glycol | 0.50 |
| Phase D | | |
| Phenoxyethanol | Phenoxyethanol | qs |
| Phase E | | |
| Potassium sorbate | Potassium Sorbate | qs |
| Phase F | | |
| $H_2o$ | Water | 5.00 |
| NaOH 30 % | Sodium Hydroxide | 0.75 |
| Phase G | | |
| Suspension according to the invention as prepared in Example 1 | - | 3.00 |

Protocol: Let phase A swell without stirring for 30 minutes and heat it to 75°C in a water bath. Heat phase B to 75°C in a water bath, while mixing well. Melt phase C and mix. Mix phase D in phase C, previously cooled. Pour phase C+D into phase A, stirring with a rotor stator mixer v=500 rpm. Mix well. Add phase B to the previous phase, stirring with a rotor stator mixer v=1000 rpm. Add phase E extemporaneously, in the previous phase under agitation mixer with rotor stator v=1000 rpm. Mix well. Add phase F, mix well.

Examples of ingredients that can be added to this formulation (marketed by SEDERMA): An ingredient comprising one or more anti-aging peptides such as Matrixyl3000®, a moisturizing ingredient such as OptimHyal®, and/or an ingredient protecting against the harmful effects of blue light like Synchrolife®.

### IN-VIVO STUDY

The aim of this study was to show the efficacy of a composition according to the invention on wrinkles.

The study was conducted on 28 female volunteers (mean age: 61 years old [55-68]) with crow's feet wrinkles, for 2 months.

Volunteers applied a cream according to the invention (the cream of the galenical part of Table 5) or a placebo cream (same formulation without the active ingredient according to the invention) twice a day on half-face.

Wrinkle parameters (depth and volume) were measured after 1 month and 2 months of application using fringe projection analysis (PRIMOS®).

The study showed a good skin tolerance of the cream according to the invention. No skin irritation related to the product creams was observed.

After 2 months of application, the cream according to the invention reduces the wrinkle appearance when placebo worsened it. This demonstrates that the antiwrinkle activity of retinol is maintained with the encapsulation method according to the invention; wrinkle volume and depth were decreased respectively down to -21 % and -34 % after 2 months of application.

An increase in the volume and depth of wrinkles in the case of the application of the placebo cream. This deterioration, present at 1 month, was confirmed after 2 months. In contrast, the application of the cream according to the invention limits the deterioration of the skin (wrinkle volume: -5 %, *p<0.05* and wrinkle depth: - 10.3 %, *p<0.05*) and this, significantly compared to placebo.

### Claims

1.   Encapsulation method according to which an aqueous suspension of submicron structured lipid particles is formed, at least one hydrophobic compound being encapsulated in said particles, said method comprising:

- a step of forming a mixture comprising an aqueous phase, a waxy phase, and an oily phase containing said at least one compound, the mixture further comprising one or more surfactants, and each phase being made homogeneous and liquid, if necessary, optionally by heating;
- a step of hot forming of an emulsion of said mixture;
- a step of reducing the particle size of said emulsion substantially to the submicron size of said particles; and
- a cooling step to form said suspension of structured particles,
said method being **characterized in that**:

- the ratio of the % by weight of wax: % by weight of oil is from 1:2 to 1:15,
- the following system of three surfactants is used:

- a first non-ionic surfactant (S1) having an HLB greater than or equal to 10;
- a second surfactant (S2) which is solid at room temperature, which is an ester of sorbitan and saturated C12-C24 fatty acid and having an HLB of less than or equal to 6; and
- a third surfactant (S3) which is a phospholipid;

all weight % being expressed relative to the total weight of the mixture or of the suspension.

2. Method according to claim 1, wherein the encapsulated compound is a hydrophobic vitamin or retinol.

3. Method according to anyone of the preceding claims, wherein said first surfactant comprises at least one alkylglucoside or one alkylpolyglucoside of at least one saturated C10-C16 fatty alcohol.

4. Method according to anyone of the preceding claims, wherein the second surfactant is a monoester chosen from sorbitan monostearate, sorbitan sesquistearate, sorbitan laurate, sorbitan oleate, sorbitan sesquioleate and sorbitan isostearate.

5. Method according to anyone of the preceding claims, wherein the third surfactant is chosen from phosphatidic acid, a phosphatidylcholine or lysophosphatidylcholine, a glycerophosphocholine, a phosphatidylserine or a lysophosphatidylserine, a phosphatidylethanolamine, a phosphatidylinositol or a sphingomyelin.

6. Method according to anyone of the preceding claims, wherein:

the first surfactant is added to the aqueous phase, dissolved if necessary, by heating; and/or
the second surfactant is added to the aqueous phase, dissolved if necessary, by heating; and/or
the third surfactant is added to the waxy phase, dissolved if necessary, by heating.

7. Method according to anyone of the preceding claims, wherein the wax is chosen from cetyl palmitate, glyceryl tribehenate, glyceryl stearate or tristearate or a wax of vegetable or animal origin.

8. Method according to anyone of the preceding claims, wherein the oil is formed from essentially C16-C18 fatty acid(s) or from mono-, di- or tri-glycerides of said fatty acid(s).

9. Method according to anyone of the preceding claims, wherein the percentage by weight of wax is from 0.5% to 10% relative to the total weight of the mixture or of the suspension.

10. Method according to anyone of the preceding claims, wherein the percentage by weight of oil is from 5% to 40% relative to the total weight of the mixture or of the suspension.

11. Method according to anyone of the preceding claims, wherein:

- the percentage by weight of the first surfactant is from 1% to 5% relative to the total weight of the mixture or of the suspension,
- the percentage by weight of the second surfactant is from 0.1% to 5% relative to the total weight of the mixture or of the suspension, and
- the percentage by weight of the third surfactant is from 0.1% to 2% relative to the total weight of the mixture or of the suspension.

12. Method according to anyone of the preceding claims, wherein the ratio % by weight of wax:% by weight of oil is between 1:5 and 1:12, the % by weight being expressed as a function of the total weight of the mixture or suspension.

13. Method according to anyone of the preceding claims, wherein the step of reducing the size of the particles is carried out by high pressure homogenization, or emulsification by membranes or micro-fluids.

14. Method according to anyone of the preceding claims, wherein the pH of the said suspension is adjusted to 6 to 9, if necessary, using a pH adjuster.

15. Method according to any one of the preceding claims, **characterized in that**:

- the wax lipid phase consists of substantially:

- 2.5 to 4% by weight of wax; and
- 0.5 to 1.5% by weight of phosphatidylcholine or hydrogenated phosphatidylserine;

- the oil lipid phase consists of substantially:

- 20 to 30% vegetable oil; and
- 2 to 4% retinol

- the aqueous phase substantially comprises:

- 1 to 3% by weight of lauryl glucoside or decyl glucoside; and
- 0.3 to 1.0% by weight of sorbitan monostearate, sorbitan laurate or sorbitan isostearate, all the percentages by weight being expressed according to the total weight of the mixture or the suspension, and the addition qs. to 100% by weight being provided by the weight of water.

16. Aqueous suspension of submicron structured lipid particles obtainable by the method according to anyone of claims 1 to 15.

17. Suspension according to claim 16, wherein the encapsulated hydrophobic compound is selected from retinol, tocopherol derivatives, coenzyme Q10, resveratrol, ferulic acid, ascorbic acid, lipoic acid or salicylic acid, and their derivatives.

18. Suspension according to claim 16 or 17, **characterized in that** the % by weight of encapsulated retinol relative to the % by weight of said suspension is at least 1%.

19. Suspension according to anyone of claims 16 to 18, wherein the polydispersity rate is 0.8 to 1.5 with an average particle size ranging from 0.1 to 0.6 $\mu$m.

20. Suspension according to anyone of claims 16 to 19, wherein the particles are structured substantially spherical and comprise a waxy shell.

21. Composition, in particular an anti-aging cosmetic composition, comprising as active ingredient an effective amount of a suspension according to anyone of claims 16 to 20 and a physiologically acceptable medium.

22. Use of a suspension according to anyone of claims 16 to 20 or of a composition according to claim 21, for use in a non-therapeutic cosmetic treatment of the skin and/or its appendages, orally or topically.

23. Suspension according to anyone of claims 16 to 20 or composition according to claim 21, for a therapeutic treatment.

**Patentansprüche**

1. Verkapselungsverfahren, gemäß dem eine wässrige Suspension von Lipidpartikeln mit Submikrometerstruktur gebildet wird, wobei mindestens eine hydrophobe Verbindung in den genannten Partikeln verkapselt ist, das genannte Verfahren umfassend:

- einen Schritt zum Bilden einer Mischung, die eine wässrige Phase, eine wachsartige Phase und eine ölige Phase umfasst, die die genannte, mindestens eine Verbindung enthält, wobei die Mischung ferner ein oder mehrere Tenside umfasst und jede Phase homogen und flüssig gemacht wird, falls notwendig, optional durch Erwärmen;
- einen Schritt des Warmformens einer Emulsion der genannten Mischung;
- einen Schritt des Reduzierens der Partikelgröße der genannten Emulsion im Wesentlichen auf die Submikrometergröße der genannten Partikel; und
- einen Kühlschritt zum Bilden der genannten Suspension von strukturierten Partikeln,
wobei das genannte Verfahren **dadurch gekennzeichnet ist, dass**:

    - das Verhältnis von Gew.-% von Wachs zu Gew.-% von Öl von 1:2 bis 1:15 beträgt,
    - das folgende System von drei Tensiden verwendet wird:

        - ein erstes nicht ionisches Tensid (S1), das einen HLB-Wert von größer als oder gleich 10 aufweist;
        - ein zweites Tensid (S2), das bei Raumtemperatur fest ist, das ein Ester von Sorbitan und einer gesättigten C12-C24-Fettsäure ist, und einen HLB-Wert von weniger als oder gleich 6 aufweist; und
        - ein drittes Tensid (S3), das ein Phospholipid ist;

wobei jedes Gew.-% relativ zum Gesamtgewicht der Mischung oder der Suspension ausgedrückt wird.

2. Verfahren gemäß Anspruch 1, wobei die verkapselte Verbindung ein hydrophobes Vitamin oder Retinol ist.

3. Verfahren gemäß einem der vorhergehenden Ansprüche, wobei das erste genannte Tensid mindestens ein Alkylglucosid oder ein Alkylpolyglucosid von mindestens einem gesättigten C10-C16-Fettalkohol umfasst.

4. Verfahren gemäß einem der vorhergehenden Ansprüche, wobei das zweite Tensid ein Monoester ist, ausgewählt aus Sorbitanmonostearat, Sorbitansesquistearat, Sorbitanlaurat, Sorbitanoleat, Sorbitansesquioleat und Sorbitanisostearat.

5. Verfahren gemäß einem der vorhergehenden Ansprüche, wobei das dritte Tensid ausgewählt ist aus Phosphatidsäure, einem Phosphatidylcholin oder Lysophosphatidylcholin, einem Glycerophosphocholin, einem Phosphatidylserin oder einem Lysophosphatidylserin, einem Phosphatidylethanolamin, einem Phosphatidylinositol oder einem Sphingomyelin.

6. Verfahren gemäß einem der vorhergehenden Ansprüche, wobei:

das erste Tensid der wässrigen Phase zugegeben wird, wenn notwendig durch Erwärmen gelöst wird; und/oder
das zweite Tensid der wässrigen Phase zugegeben wird, wenn notwendig durch Erwärmen gelöst wird; und/oder
das dritte Tensid der wachsartigen Phase zugegeben wird, wenn notwendig durch Erwärmen gelöst wird.

7. Verfahren gemäß einem der vorhergehenden Ansprüche, wobei das Wachs aus Cetylpalmitat, Glyceryltribehenat, Glycerylstearat oder Tristearat oder einem Wachs pflanzlichen oder tierischen Ursprungs ausgewählt ist.

8. Verfahren gemäß einem der vorhergehenden Ansprüche, wobei das Öl im Wesentlichen aus C16- bis C18-Fettsäuren oder aus Mono-, Di- oder Triglyceriden der genannten Fettsäuren gebildet wird.

9. Verfahren gemäß einem der vorhergehenden Ansprüche, wobei der Gewichtsprozentsatz des Wachses von 0,5 % bis 10 % relativ zum Gesamtgewicht der Mischung oder der Suspension beträgt.

10. Verfahren gemäß einem der vorhergehenden Ansprüche, wobei der Gewichtsprozentsatz des Öls von 5 % bis 40 % relativ zum Gesamtgewicht der Mischung oder der Suspension beträgt.

11. Verfahren gemäß einem der vorhergehenden Ansprüche, wobei:

- der Gewichtsprozentsatz des ersten Tensids von 1 % bis 5 % relativ zum Gesamtgewicht der Mischung oder der Suspension beträgt,
- der Gewichtsprozentsatz des zweiten Tensids von 0,1 % bis 5 % relativ zum Gesamtgewicht der Mischung oder der Suspension beträgt, und

- der Gewichtsprozentsatz des dritten Tensids von 0,1 % bis 2 % relativ zum Gesamtgewicht der Mischung oder der Suspension beträgt.

12. Verfahren gemäß einem der vorhergehenden Ansprüche, wobei das Verhältnis von Gew.-% Wachs zu Gew.-% Öl zwischen 1:5 und 1:12 beträgt, wobei die Gew.-% als Funktion des Gesamtgewichts der Mischung oder Suspension ausgedrückt sind.

13. Verfahren gemäß einem der vorhergehenden Ansprüche, wobei der Schritt des Reduzierens der Größe der Partikel durch Hochdruckhomogenisierung oder Emulgierung durch Membranen oder Mikrofluide durchgeführt wird.

14. Verfahren gemäß einem der vorhergehenden Ansprüche, wobei der pH der genannten Suspension bei Bedarf unter Verwendung eines pH-Einstellers auf 6 bis 9 eingestellt wird.

15. Verfahren gemäß einem der vorhergehenden Ansprüche, **dadurch gekennzeichnet, dass**:

   - die Wachs-Lipid-Phase im Wesentlichen besteht aus:

      - 2,5 bis 4 Gew.-% Wachs; und
      - 0,5 bis 1,5 Gew.-% Phosphatidylcholin oder hydriertem Phosphatidylserin;

   - die Öl-Lipid-Phase im Wesentlichen besteht aus:

      - 20 bis 30 % Pflanzenöl; und
      - 2 bis 4 % Retinol

   - die wässrige Phase im Wesentlichen umfasst:

      - 1 bis 3 Gew.-% Laurylglucosid oder Decylglucosid; und
      - 0,3 bis 1,0 Gew.-% Sorbitanmonostearat, Sorbitanlaurat oder Sorbitanisostearat,

   wobei alle Gewichtsprozente gemäß dem Gesamtgewicht der Mischung oder Suspension ausgedrückt sind, und die Ergänzungsmenge auf 100 Gew.-% durch das Gewicht des Wassers bereitgestellt wird.

16. Wässrige Suspension von Lipidpartikeln mit Submikrometerstruktur, erhältlich durch das Verfahren gemäß einem der Ansprüche 1 bis 15.

17. Suspension gemäß Anspruch 16, wobei die verkapselte hydrophobe Verbindung ausgewählt ist aus Retinol, Tocopherolderivaten, Coenzym Q10, Resveratrol, Ferulasäure, Ascorbinsäure, Liponsäure oder Salicylsäure und ihren Derivaten.

18. Suspension gemäß Anspruch 16 oder 17, **dadurch gekennzeichnet, dass** die Gew.-% von verkapseltem Retinol relativ zu den Gew.-% der genannten Suspension mindestens 1 % beträgt.

19. Suspension gemäß einem der Ansprüche 16 bis 18, wobei der Polydispersitätsindex 0,8 bis 1,5 beträgt und die durchschnittliche Partikelgröße im Bereich von 0,1 bis 0,6 $\mu$m liegt.

20. Suspension gemäß einem der Ansprüche 16 bis 19, wobei die Partikel im Wesentlichen kugelförmig strukturiert sind, und eine wachsartige Hülle umfassen.

21. Zusammensetzung, insbesondere eine Anti-Aging-Kosmetikzusammensetzung, die als Wirkstoff eine wirksame Menge einer Suspension gemäß einem der Ansprüche 16 bis 20 und ein physiologisch verträgliches Medium umfasst.

22. Verwendung einer Suspension gemäß einem der Ansprüche 16 bis 20 oder einer Zusammensetzung gemäß Anspruch 21 zur Verwendung für eine nicht therapeutische kosmetische Behandlung der Haut und/oder ihrer Anhangsgebilde, oral oder topisch.

23. Suspension gemäß einem der Ansprüche 16 bis 20 oder Zusammensetzung gemäß Anspruch 21 für eine therapeutische Behandlung.

**Revendications**

1. Procédé d'encapsulation selon lequel une suspension aqueuse de particules lipidiques structurées submicroniques est formée, au moins un composé hydrophobe étant encapsulé dans lesdites particules, ledit procédé comprenant :

- une étape de formation d'un mélange comprenant une phase aqueuse, une phase cireuse et une phase huileuse contenant ledit au moins un composé, le mélange comprenant en outre un ou plusieurs tensio-actifs, et chaque phase étant rendue homogène et liquide le cas échéant optionnellement par chauffage ;
- une étape de formation à chaud d'une émulsion dudit mélange ;
- une étape de réduction de la taille des particules de ladite émulsion sensiblement à la taille submicronique desdites particules ; et
- une étape de refroidissement de manière à former ladite suspension de particules structurées,

ledit procédé étant **caractérisé en ce que** :

- le ratio de % en poids de cire : % en poids d'huile est compris entre 1:2 et 1:15,
- le système des trois tensio-actifs suivant est utilisé :

  - un premier tensio-actif non ionique (S1) présentant une HLB supérieure ou égale à 10 ;
  - un deuxième tensio-actif (S2) qui est solide à température ambiante et qui est un ester de sorbitane et d'acide gras saturé en C12-C24 et présentant une HLB inférieure ou égale à 6 ; et
  - un troisième tensio-actif (S3) qui est un phospholipide ;

tous les % en poids étant exprimés par rapport au poids total du mélange ou de la suspension.

2. Procédé selon la revendication 1, dans lequel le composé encapsulé est une vitamine hydrophobe ou le rétinol.

3. Procédé selon quelconque l'une des revendications précédentes, dans lequel ledit premier tensio-actif comprend au moins un alkylglucoside ou un alkylpolyglucoside d'au moins un alcool gras saturé en C10-C16.

4. Procédé selon l'une quelconques des revendications précédentes, dans lequel le deuxième tensio-actif est un monoester choisi parmi le monostéarate de sorbitane, le sesquistéarate de sorbitane, le laurate de sorbitane, l'oléate de sorbitane, le sesquioléate de sorbitane et l'isostéarate de sorbitane.

5. Procédé selon l'une quelconque des revendications précédentes, dans lequel le troisième tensio-actif est choisi parmi l'acide phosphatidique, une phosphatidylcholine ou lysophosphatidylcholine, une glycérophosphocholine, une phosphatidylsérine ou une lysophosphatidylsérine, une phosphatidyléthanolamine, un phosphatidylinositol ou une sphingomyéline.

6. Procédé selon l'une quelconque des revendications précédentes, dans lequel le premier tensio-actif est ajouté à la phase aqueuse, solubilisé, le cas-échéant, par chauffage ; et/ou

le deuxième tensio-actif est ajouté à la phase aqueuse, solubilisé, le cas-échéant, par chauffage ; et/ou
le troisième tensio-actif est ajouté à la phase cireuse, solubilisé, le cas échéant, par chauffage.

7. Procédé selon l'une quelconque des revendications précédentes, dans lequel la cire est choisie parmi le palmitate de cétyle, le tribéhénate de glycéryl, le stéarate ou le tristéarate de glycéryl ou une cire d'origine végétale ou animale.

8. Procédé selon l'une quelconque des revendications précédentes, dans lequel l'huile est formée d'acide(s) gras essentiellement en C16-C18 ou de mono-, di- ou tri-glycérides desdits acide(s) gras.

9. Procédé selon l'une quelconque des revendications précédentes, dans lequel le pourcentage en poids de cire est de 0,5% à 10% par rapport au poids total du mélange ou de la suspension.

10. Procédé selon l'une quelconque des revendications précédentes, dans lequel le pourcentage en poids d'huile est de 5% à 40% par rapport au poids total du mélange ou de la suspension.

11. Procédé selon l'une quelconque des revendications précédentes, dans lequel :

   - le pourcentage en poids du premier tensio-actif est de 1% à 5% par rapport au poids total du mélange ou de la suspension,
   - le pourcentage en poids du deuxième tensio-actif est de 0,1% à 5% par rapport au poids total du mélange ou de la suspension, et
   - le pourcentage en poids du troisième tensio-actif est de 0,1% à 2% par rapport au poids total du mélange ou de la suspension.

12. Procédé selon l'une quelconque des revendications précédentes, dans lequel le ratio % en poids de cire : % en poids d'huile est compris entre 1:5 et 1:12, les % en poids étant exprimés en fonction du poids total du mélange ou de la suspension.

13. Procédé selon l'une quelconque des revendications précédentes, dans lequel l'étape de réduction de la taille de particules est effectuée par homogénéisation sous-haute pression ou par émulsification par membranes ou par micro-fluides.

14. Procédé selon l'une quelconque des revendications précédentes, dans lequel le pH de ladite suspension est ajusté à 6 à 9 le cas échéant à l'aide d'un ajusteur de pH.

15. Procédé selon l'une quelconque des revendications précédentes, **caractérisé en ce que** :

   - la phase lipidique cireuse est constituée de sensiblement :

      - 2,5 à 4 % en poids de cire ; et
      - 0,5 à 1,5 % en poids de phosphatidylcholine ou de phosphatidylsérine hydrogénée ;

   - la phase lipidique huileuse est constituée de sensiblement :

      - 20 à 30 % d'huile végétale ; et
      - 2 à 4% de rétinol

   - la phase aqueuse comprend sensiblement :

      - 1 à 3 % en poids de lauryl glucoside ou décyl glucoside ; et
      - 0,3 à 1,0 % en poids de monostéarate de sorbitane, laurate de sorbitane ou isostéarate de sorbitane ;

   tous les pourcentages en poids étant exprimés en fonction du poids total du mélange ou de la suspension, et le complément qsp à 100% en poids étant apporté par le poids d'eau.

16. Suspension aqueuse de particules lipidiques structurées submicroniques susceptible d'être obtenue par le procédé selon l'une quelconque des revendications 1 à 15.

17. Suspension selon la revendication 16, **caractérisée en ce que** le composé hydrophobe encapsulé est choisi parmi le rétinol, les dérivés du tocophérol, la coenzyme q10, le resvératrol, l'acide férulique, l'acide ascorbique, l'acide lipoïque ou l'acide salicylique, et leurs dérivés.

18. Suspension selon la revendication 16 ou 17, **caractérisée en ce que** le % en poids de rétinol encapsulé par rapport au % en poids de ladite suspension est d'au moins 1%.

19. Suspension selon l'une des revendications 16 à 18, **caractérisée en ce que** le taux de polydispersité est de 0,8 à 1,5 avec une taille moyenne de particules allant de 0,1 à 0,6 $\mu$m.

20. Suspension selon l'une quelconque des revendications 16 à 19, **caractérisée en ce que** les particules sont structurées sensiblement sphériques et comportent une enveloppe cireuse.

**21.** Composition, notamment cosmétique antiâge, comprenant à titre d'ingrédient actif une quantité efficace d'une suspension selon l'une quelconque des revendications 16 à 20 et un véhicule physiologiquement acceptable.

**22.** Utilisation d'une suspension selon l'une quelconque des revendications 16 à 20 ou d'une composition selon la revendication 21, pour un traitement cosmétique non-thérapeutique de la peau et/ou de ses phanères, par voie orale ou topique.

**23.** Suspension selon l'une quelconque des revendications 16 à 20 ou composition selon la revendication 21, pour un traitement thérapeutique.

[Fig.1]

S 1

Wax + S2 + S3

Retinol + oil

[Fig.2]

[Fig.3]

[Fig.4]

[Fig.5]

[Fig.6]

[Fig.7]

[Fig.8]

[Fig.9]

[Fig.10]

[Fig.11]

[Fig.12]

[Fig.13]

Donor chamber

Skin membrane

Receptor chamber

Magnetic stirrer bar

Sampling port

Receptor fluid

[Fig.14]

[Fig.15]

[Fig.16]

[Fig.17]

[Fig.18]

[Fig. 19]

Without product, t = 8h

[Fig. 20]

Without product, t = 24h

[Fig.21]

Free retinol, t = 8h

[Fig.22]

Free retinol, t = 24h

[Fig. 23]

Encapsulated retinol, t = 8h

[Fig.24]

Encapsulated retinol, t = 24h

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2047838 A
- WO 2015181688 A
- WO 2014080376 A
- FR 1850845

**Non-patent literature cited in the description**

- **J. PARDEIKE et al.** Lipid nanoparticles (SLN, NLC) in cosmetic and pharmaceutical dermal products. *International Journal of Pharmaceutics*, 2009, vol. 366, 170-184
- **F. OLECHOWSKI et al.** BergaCare SmartLipids: concentrated commercial lipophilic active to improve the performance of dermal products. *Beilstein J. Nanotechnol.*, 2019, vol. 10, 2152-2162
- **SEUNG-HYUN JUN et al.** Synthesis of lipid nano-carrier loaded with retinol via vacuum emulsification to improve topical delivery. *Polymers*, 2021, vol. 13, 826
- **PORNTHIDA RIANGJANAPATEE et al.** Effect of the surfactant on nanostructured lipid carriers loaded with lycopene. *Drug Discoveries & Therapeutics*, 2012, vol. 6 (3), 163-168
- **APOSTOLOU et al.** *Journal of Pharmaceutical Sciences*, 2021, vol. 110, 2859-2872
- International cosmetic ingredient dictionary & hand-book. The Personal Care Products Council. Cosmetic, Toiletry, and Fragrance Association, Inc.'', Washington, D.C